# EUROPEAN PATENT APPLICATION

(11) **EP 2 053 059 A1**
(43) Date of publication of application: **29.04.2009**
(21) Application number: 07291300.7
(22) Date of filing: 26.10.2007
(51) Int. Cl.: C07K 14/47, C07K 14/705

(54) **Complexes of TRPC domains and SESTD1 domains and methods and uses involving the same**

(71) Applicant: Sanofi-Aventis, 75013 Paris (FR)
(72) Inventor: The designation of the inventor has not yet been filed
(74) Representative: Schneider, Rudolf

(57) **Abstract**

The present invention relates to an isolated complex comprising a first protein comprising or consisting of a classical transient receptor potential channel (TRPC) or a functionally active variant thereof and a second protein comprising or consisting of the first spectrin (Spec 1) domain of SEC14 and spectrin domains 1 (SESTD1), a test system comprising the first protein and the second protein as well as means for detecting interaction of the first and the second protein, a method for screening for a TRPC modulator using the system and the use of the test system for the identification of a TRPC modulator.

## Description

The present invention relates to an isolated complex comprising a first protein comprising or consisting of a transient receptor potential channel (TRPC) or a functionally active variant thereof and a second protein comprising or consisting of the first spectrin (Spec 1) domain of SEC14 and spectrin domains 1 (SESTD1), a test system comprising the first protein and the second protein as well as means for detecting interaction of the first and the second protein, a method for screening for a TRPC modulator using the system and the use of the test system for the identification of a TRPC modulator.

Classical transient receptor potential channel proteins (TRPCs) constitute a family of nonspecific cation channels. They were the first found mammalian homologues of *Drosophila* TRP and are the subfamily closest related to its well characterized invertebrate ancestor. Despite these facts, there are many open questions regarding their native composition and activation mechanisms, physiological functions, and roles in pathophysiology and disease.

*In situ* identification of native TRPC channels is complicated by their wide and partially overlapping distribution, potential heteromultimerization, similar electrophysiological properties and a paucity of tool compounds to unequivocally trace these channels. Known organic inhibitors, e.g. 2-aminoethoxydiphenyl borate (2-APB), econazole, flufenamate, ruthenium red, SK&F 96365, and inorganic blockers, e.g. Gd³⁺, La³⁺, are not potent and specific enough. Studies in transgenic mice already have proven to be valuable in unravelling possible physiological functions of certain TRPCs. However, these model systems do not exist for each of the seven mammalian TRPC channels so far, their generation and analysis is time- and cost-consuming and they also have limitations as they are vulnerable to compensatory effects by closely related channels. Studies with dominant negative channel subunits or gene disruption with small interfering RNA (siRNA) depend on induction or suppression, respectively, of protein expression. As this might take some time, compensatory effects are possible as well, which are not expected to be seen when channels are instantaneously blocked with a selective tool compound.

The importance and value of discriminating tool compounds is underscored by the rapid development of TRPV1-targeting drugs. This channel also belongs to the TRP superfamily and its physiological function was unravelled by the use of a specific activator (capsaicin). Ten years after its cloning, several clinical trials are ongoing and test the therapeutic value of TRPV1 modulation in a number of indications, e.g. in different types of pain, migraine, and urinary urge incontinence.

Dysregulation of endothelial calcium signaling is involved in many cardiovascular pathological effects, such as atherosclerosis, coronary syndrome, heart and renal failure, hypertension and thrombosis. Evidence from TRPC4-deficient mice suggests its necessity for agonist-induced endothelium-dependent vascular relaxation and its involvement in regulating endothelial barrier function. Therefore, modulating TRPCs, such as TRPC4, may be a promising approach to treat the aforementioned pathophysiological conditions. However, drug discovery for TRPCs is hampered by difficulties to faithfully reconstitute native currents in heterologous expression systems.

Accordingly, one object of the present invention was to develop new pharmacological tools that may be used to gain a better understanding of TRPC channel function in cells and beyond, and to identify novel regulators or modulators of TRPC channel complexes. Preferably, the new tools provide for economic and convenient testing of substances, such as high throughput screening methods.

The object of the present invention was solved by providing an isolated complex comprising
- a first protein comprising or consisting of a transient receptor potential channel (TRPC) or a functionally active variant thereof and
- a second protein comprising or consisting of the first spectrin (Spec 1) domain of SEC14 and spectrin domains 1 (SESTD1).

An alternative isolated complex comprises
- a first protein comprising or consisting of a domain of a transient receptor potential channel (TRPC) or a functionally active variant thereof, the domain having the sequence LXXXXXYQEVXRNLVKRYVAAMIRXXKT (SEQ ID NO:1), wherein each X represents any amino acid residue and
- a second protein comprising or consisting of the Spec 1 domain of SESTD1.
   Accordingly, a first and second aspect of the present invention relates to an isolated complex comprising
- a first protein comprising or consisting of a transient receptor potential channel (TRPC) or a functionally active variant thereof and
- a second protein comprising or consisting of the first spectrin (Spec 1) domain of SEC14 and spectrin domains 1 (SESTD1)
or an isolated complex comprising
- a first protein comprising or consisting of a domain of a transient receptor potential channel (TRPC) or a functionally active variant thereof, the domain having the sequence LXXXXXYQEVXRNLVKRYVAAMIRXXKT (SEQ ID NO:1), wherein each X represents any amino acid residue and
- a second protein comprising or consisting of the Spec 1 domain of SESTD1.

An isolated complex in the context of the present invention relates to a complex of the first and the second protein as defined herein which is not in its natural environment. Accordingly, the "isolated complex" is not associated with proteins not being part of the signal transduction of the TRPC and the SESTD1 as defined herein or it (or its components) is isolated from a cell in which it normally occurs or is isolated from a cell in which the nucleic acid coding for the same has been expressed or is essentially free from other proteins from the same cellular source not involved in the respective signal transduction pathway. The complex may be a naturally occurring complex, preferably a naturally occurring complex of TRPC and SESTD1 or part thereof (as defined herein). However, the proteins of the complex may also be artificial in that they do not naturally occur, in that they may encompass one or more sections which are naturally not connected to each other, for example, a fusion protein comprising or consisting of a part of TRPC or SESTD1 as defined herein and a further protein such as a second domain or other component used for, e.g., detection and/or purification purposes.

Preferably, the term "isolated complex" means a protein complex which is essentially separated from other cellular components of its natural environment. However, after isolation of the proteins of the complex, cellular components may be added again, e.g., for measuring signal transduction pathways. Additionally, the skilled person will understand that the isolated complex is to be kept under suitable conditions allowing activity of the isolated complex, e.g., suitable buffers, pH values, ions, etc.

The first protein of the complex of the invention comprises or consists of:
- in a first alternative a transient receptor potential channel (TRPC) or a functionally active variant thereof
   or
- in a second alternative a domain of a transient receptor potential channel (TRPC) or a functionally active variant thereof, the domain having the sequence LXXXXXYQEVXRNLVKRYVAAMIRXXKT (SEQ ID NO:1), wherein each X represents any amino acid residue.

The TRPC may be any naturally occurring TRPC. Based on amino acid sequence homology and functional similarities TRPCs can be subclassified into four groups. Being quite unique within the TRPC family, TRPC1 and TRPC2 each constitute a subfamily by themselves while TRPC4 and -5 are merged just as TRPC3, -6, and -7. Heteromeric interactions within these subfamilies have been shown as well as coassembly of TRPC1 with either TRPC4/5- or TRPC3/6/7-subfamily members. It was long thought that no cross-association occurs between the TRPC4/5 and TRPC3/6/7 subgroups, but recently an endogenous redox-sensitive TRPC3/4 heteromer has been reported in porcine aortic endothelial cells.

The seven TRPCs will be introduced with their possible *in vivo* functions below.

*TRPC1 subfamily:* Investigation of the broadly expressed homomeric TRPC1 has been hampered by absent plasma membrane targeting of the ectopic protein in cell lines. Depending on the overexpression system used, reports range from lack of robust TRPC1 signals due to retention in intracellular membranes to detailed description of channel properties in sf9 cells. Possible explanations are the absence of auxiliary subunits or interacting proteins in overexpression systems. Plasma membrane expression of TRPC1 has been shown to depend on interaction with other proteins, e.g. TRPCs, caveolin-1 and RhoA. Also in sf9 cells it is not certain whether homo- or heteromeric or even native channels, which could be stimulated by TRPC1, are measured. So far, ectopically expressed homomeric TRPC1 was described as receptor-, DAG-, IP₃R- or stretch-activated channel or as a non-functional channel subunit and the existence of a native TRPC1 homomer was not unequivocally proven so far. But the endogenous protein is required for the excitatory postsynaptic conductance (EPSC) in Purkinje cells therefore it could be involved in neuronal plasticity. Moreover, TRPC1 is up-regulated in neointimal hyperplasia and cardiac hypertrophy and its function could be affected in patients suffering from Duchenne muscular dystrophy. Recently, a role for TRPC1 was proposed in breast cancer. TRPC1 has also been reported to interact with TRPP2, a distantly related TRP protein involved in development of polycystic kidney disease. In conclusion, TRPC1 seems to be engaged in many patho- and physiological processes and some of its functional roles are not easily compensated by related TRPCs. These characteristics along with its accessibility to blocking extracellular antibodies make it a potential drug target, but it is probably not easily applicable due to its broad expression and favoured heteromultimerization.

*TRPC2 subfamily:* TRPC2 is a pseudogene in humans but essential for pheromone sensation in rodents. Male mice lacking this channel do not show typical male-male aggressive behaviour and even court consexuals. Antibodies directed to an extracellular domain of TRPC2 inhibit the acrosomal reaction pointing towards its importance in fertilization. However, TRPC2 knock-out mice showed no defects in reproduction. TRPC2 does not seem to heteromultimerize with other TRPC channels.

*TRPC3*/*6*/*7 subfamily:* Its members share 70 - 80 % amino acid identity and are directly activated by the PLC product DAG. TRPC3 and -6 activities are regulated by N-glycosylation and phosphorylation through the non-receptor tyrosine kinases Src and Fyn.

In humans TRPC3 is highly expressed in brain, smooth muscle and vascular endothelial cells. It seems to be involved in axon growth guidance, synaptic plasticity around the time of birth and cardiac Ca²⁺ homeostasis. In cardiomyocytes, abnormal accumulation of intracellular Na⁺ levels due to TRPC3 has been shown to reverse the Na⁺/Ca²⁺ exchanger (NCX1) mode. This mode reversal transports Ca²⁺ into the cell and might be involved in pathophysiological processes, e.g. heart failure and ischemia. TRPC3 was found to coassemble with TRPC4 into a redox-sensitive channel. It is hypothesized that oxidative stress in brain, for example induced by stroke, could activate redox-sensitive TRPC channels leading to uncontrolled Ca²⁺ influx. The resulting neurodegeneration could eventually be minimized by TRPC antagonists.

TRPC6 is present both in vascular and airway smooth muscle cells (SMC) and seems to play essential roles in the vascular and cardiac system. Its stimulation by agonists or increasing intravascular pressure is postulated to depolarize the membrane. Subsequently, L-type voltage-gated Ca²⁺ channels are activated which finally mediate muscle contraction and reflex vasoconstriction (Bayliss effect). On the contrary, agonist-induced bronchoconstriction mainly depends on Ca²⁺ influx mediated by voltage-independent channels (such as TRPC6), hence, L-type Ca²⁺ channel blockers are not effective, e.g. in asthma and chronic obstructive pulmonary disease (COPD). Pulmonary artery SMC (PASMC) of patients suffering from idiopathic pulmonary arterial hypertension (IPAH) are hyperproliferative and TRPC6 (and TRPC3) expression is significantly increased. PASMC hyperproliferation was markedly reduced after treatment with TRPC6 small-interferring RNA (siRNA) or bosentan, an endothelin receptor blocker approved for the treatment of IPAH patients. TRPC6 is also found in leukocytes probably mediating inflammatory responses in asthma and COPD. Therefore, TRPC6 inhibition seems to be an interesting therapeutic strategy for the treatment of IPAH, a progressive, life-shortening disease (right heart failure), and other chronic respiratory diseases. But TRPC6 also has physiological functions in airway SMC that should rather not be blocked. It is necessary to regulate acute hypoxic pulmonary vasoconstriction (HPV). Blood flow is directed from poorly to well ventilated areas maintaining proper gas exchange under acute hypoxic conditions. Disturbances in HPV as occurring in pneumonia, the adult respiratory distress syndrome, and liver failure, can cause life-threatening hypoxemia. TRPC6 is essential in HPV but not involved in pulmonary vascular hypertension and remodeling resulting from chronic hypoxia. Convincing evidence for TRPC6 involvement in hereditary focal segmental glomerulosclerosis (FSGS), a significant cause of end-stage renal disease, has also been presented. TRPC6 gain-of-function mutants lead to increased Ca²⁺ influx into podocyte foot processes, but it is not known whether and how this is disease-causing. These cells together with the glomerular slit diaphragm form a crucial part of the glomerular filter whose damage results in proteinuria. The glomerular filter is disturbed in transgenic mice deficient of nephrin, a vital slit diaphragm component. TRPC6 is overexpressed and mislocated in podocytes of these mice. Recently, it was also shown *in vitro* that TRPC6 expression is up-regulated in complement-treated podocytes leading to actin cytoskeleton rearrangement, whereas transient channel overexpression *in vivo* led to proteinuria in mice. TRPC6 deficient mice have an unexpected and surprising phenotype. Contrary to the proposed physiological functions of the channel described above, these animals showed airway smooth muscle hyperreactivity in response to bronchoconstrictors, an elevated mean arterial blood pressure that could be further increased by inhibition of nitric oxide (NO) synthase, and a lowered threshold of the intravascular pressure necessary to induce the Bayliss effect (exaggerated reflex vasoconstriction). Also the basal and agonist-induced cation entry in SMC of TRPC6^{-/-} mice is higher. Partly, this can be explained by an increased expression of a closely related channel, TRPC3. It has a higher basal activity, is less tightly regulated by vasoconstrictors and has consequently overcompensated TRPC6 knock-out, demonstrating that both channels are not functionally redundant. The opposite approach revealed a role for TRPC6 in the pathogenesis of cardiac hypertrophy. Cardiac specific TRPC overexpression in transgenic mice leads to increased Ca²⁺ influx that couples via calcineurin to nuclear factor of activated T cells (NFAT) stimulation. Pathological heart remodelling was accelerated and these mice had a shortened life expectancy. Whereas *in vivo* TRPC6 upregulation in cardiomyocytes participates in hypertrophy, it seems to have protective antifibrotic functions in cardiac fibroblasts *in vitro*. Further *in vivo* studies are needed to estimate the therapeutic value of TRPC6 modulation and the involvement of TRPC3 and TRPC3/6 heteromers in the pathogenesis of heart failure.

TRPC7 is expressed in eye, heart and lung and lower transcript levels are found in brain, spleen and testis but its physiological functions remain obscure.

*TRPC4*/*5 subfamily:* These channels share 64% identity and are most closely related to TRPC1 (persuading some groups to classify TRPC1 within this subfamily). A unique feature among TRP and other store-operated channels is their potentiation by lanthanides (e.g. Gd³⁺, La³⁺) after G_{q/11}-receptor mediated activation. In contrast to the TRPC3/6/7 subgroup they are not directly activated by DAG application but several endogenous TRPC5 activators were identified recently: lysophosphatidylcholine (LPC) and sphingosine 1-phosphate (S1 P). S-nitrosylation, e.g. by nitric oxide (NO), has been shown to activate both TRPC4 and TRPC5.

TRPC4 was the first TRP gene to be knocked out in mice. It is widely expressed and found in endothelial and smooth muscle cells. TRPC4^{-/-} mice are viable and reach maturation. However, agonist-induced Ca²⁺ entry into endothelial cells of TRPC4^{-/-} mice is markedly reduced resulting in decreased endothelium-dependent vasorelaxation. Further studies were performed with thrombin, an important inflammation mediator that is involved in the pathogenesis of vascular injury. In lungs, it increases vascular permeability and thus tissue water content. Lung EC of TRPC4^{-/-} mice lack thrombin-induced actin stress fiber formation, cell retraction is impaired, and lung microvascular permeability subsequently reduced by about 50%. TRPC4 is also expressed in different cells within the central nervous system and seems to be involved in neurotransmitter release. In F2 terminals of thalamic interneurones from TRPC4^{-/-} mice release of γ-aminobutyric acid (GABA) following application of 5-hydroxytryptamin (5-HT, serotonin) is drastically reduced. In contrast, GABA release upon stimulation of metabotropic glutamate receptors is not changed. The thalamus regulates sleep and wakefulness and TRPC4 could participate in processing of visual information depending on the sleep/wake cycle. TRPC4 expressed in pancreatic β-islets could be involved in insulin secretion, but glucose-tolerance test results were similar in wild-type and TRPC4-deficient mice. Finally, the channel seems to be involved in regulating the motility of the gastrointestinal tract by modulating the pacemaker activity of interstitial cells of Cajal (ICC).

TRPC5 is highly enriched in brain but also found peripheral, e.g. in SMC. In humans, the gene is located on a region of the X chromosome associated with non-syndromic mental retardation. TRPC5 homomers expressed in growth cones of rat hippocampal neurons regulate neurite outgrowth as well as growth cone morphology. Functional channel suppression by transfection of a dominant-negative mutant led to abnormally prolonged neurites whereas overexpression resulted in neurite outgrowth inhibition. Phosphatidyl-inositiol 4-phosphate 5-kinase (PIP(5)Kα)-dependent channel insertion from vesicles into the plasmamembrane was demonstrated to be crucial for neurite length regulation by TRPC5. TRPC5 could also be multifunctionally important within the cardiovascular system. A dominant-negative TRPC5 mutant inhibited cell motility evoked by sphingosine 1-phosphate (a TRPC5 activator) of vascular smooth muscle cells, the same effect was accomplished with an anti-TRPC5 antibody. SMC motility is crucial in physiological adaptive processes like wound healing but is also involved in inflammatory occlusive diseases, e.g. atherosclerosis. In monocytes from hypertensive patients increased TRPC5 expression and channel-mediated Ca²⁺ influx was reported. Furthermore, compensatory upregulation of TRPC5 protein expression and activity (as weii as of TRPC4, NCX and several transcription factors) was observed by siRNA mediated downregulation of the Ca²⁺-ATPase SERCA2 in neonatal rat cardiac myocytes. In human failing heart from patients with end-stage idiopathic dilated cardiomyopathy TRPC5 was even selectively upregulated, whereas TRPC3 mRNA and protein was not detectable and TRPC1, -4, and -6 expression levels were not altered. On the basis of these data, involvement of TRPC5 (and TRPC4) in cardiac hypertrophy is conceivable.

In accordance with the present invention, the first protein may also comprise or consists of a functionally active variant of any of the TRPCs as defined herein. The functionally active variant of the TRPC may be derived from the TRPC by one or more amino acid additions, deletions or substitutions, wherein the variant is still capable of binding to a natural occurring SESTD1. Preferably, the variant is a fragment of the TRPC still comprising a SESTD1 binding domain providing for the capability of binding to the SESTD1 and/or Spec 1 of SESTD1. The binding of the variant to SESTD1 may be examined as described in Examples 1, 2, 3, 4, 5 or 6.

Preferably, the affinity (K_{D} = k_{off}/kₒₙ) of the functionally active variant to the SESTD1 amounts to at least about 0.1 K_{D} of the naturally occurring TRPC, especially TRPC4 or TRPC5, more preferably to least about 0.3 K_{D}, still more preferably to least about 0.5 K_{D}, even more preferably to least about 0.7 K_{D} and most preferably to least about 1 K_{D}. The affinity of a protein for another molecule may be determined by a variety of standard techniques including binding studies using a labeled marker such as a radioligand and fluorescence marker etc and is well known to the skilled practitioner.

The functionally active variant may be a fragment of the any naturally occurring TRPC. Alternatively, the functionally active variant may also be a chimeric TRPC comprising domains or segments of two or more different TRPCs. The functionally active variant may also encompass one or more segments with mutations (e.g. substitutions, additions and/or deletions) which may alter the binding properties of the variant to the TRPC. However, the variant is defined in that it is still capable of binding to a natural occurring SESTD1 and/or Spec 1 of SESTD1.

In one embodiment of the invention the functionally active variant is defined in that it is a TRPC variant comprising or consisting of a sequence homologous to SEQ ID NO:1, SEQ ID NO:2 or SEQ ID NO:3. Preferably, this sequence is a fragment of a naturally occurring TRPC. The sequences of various TRPC proteins are derivable from protein databases such as NCBi (National Center for Biotechnology information) and homologous sequences may be identified by suitable alignment programs as known to the skilled practitioner.

In one embodiment of the present invention, the first protein of the present invention comprises a marker, particularly a tag.

A marker in the context of the present invention may be any kind of molecule which can be easily detected. In the present invention, the molecule is bound to TRPC or a functionally active variant thereof, therefore, the presence of the marker is indicative for the presence of the first protein. Markers (also referred to as labels) are known to a skilled person and include, for example, radiolabels (such as ³H, ³²P, ³⁵S or ¹⁴C), fluorescence markers (such as fluorescein, green fluorescence protein, yellow fluorescence protein or DyLight 488), enzymes (such as horseradish peroxidase, β-lactamase, alkaline phosphatase or β-glucosidase) or an antigen-detectable by a suitable antibody or antibody fragment.

Preferably, the marker is a tag. Tags are usually peptides or proteins which are used as biochemical indicators. They may be included into a protein, such as a recombinant, expressed protein and can serve several purposes. Preferably, they are used for purifying the proteins to which they are attached using standard conditions suitable for the particular tag. However, the tags may be also used as indicators in order to detect the presence of a particular protein.

A number of (affinity) tags are known at present. These are usually divided into 3 classes according to their size: small tags have a maximum of 12 amino acids, medium-sized ones have a maximum of 60 and large ones have more than 60. The small tags include the Arg-tag, the His-tag, the Strep-tag, the Flag-tag, the T7-tag, the V5-peptide-tag and the c-Myc-tag, the medium-sized ones include the S-tag, the HAT-tag, the calmodulin-binding peptide, the chitin-binding peptide and some cellulose-binding domains. The latter can contain up to 189 amino acids and are then regarded, like the GST- (glutathione-S-transferase-) and MBP-tag (maltose binding protein-tag), as large affinity tags.

In order to produce especially pure proteins, so-called double tags or tandem tags were developed. In this case the proteins are purified in two separate chromatography steps, in each case utilizing the affinity of a first and then of a second tag. Examples of such double or tandem tags are the GST-His-tag (glutathione-S-transferase fused to a polyhistidine-tag), the 6xHis-Strep-tag (6 histidine residues fused to a Strep-tag), the 6xHis-tag100-tag (6 histidine residues fused to a 12-amino-acid protein of mammalian MAP-kinase 2), 8xHis-HA-tag (8 histidine residues fused to a haemagglutinin-epitope-tag), His-MBP (His-tag fused to a maltose-binding protein), FLAG-HA-tag (FLAG-tag fused to a hemagglutinin-epitope-tag), and the FLAG-Strep-tag.

Preferably, the first protein of the present invention comprises a tag selected from the group consisting of His-tag, Arg-tag, Strep-tag, Flag-tag, T7-tag, V5-peptide-tag, c-Myc-tag, S-tag, HAT-tag, calmodulin-binding peptide-tag, chitin-binding peptide-tag, GST-tag and MBP-tag. However, any other tag may be also used, but some tags such as His-tag, Arg-tag, Strep-tag, Flag-tag or GST-tag are preferred. Suitable examples for TRPCs or variants thereof encompassing a tag are also shown in the Examples.

In an embodiment of the invention the first protein comprises a marker or tag, wherein the marker or tag is removable from the protein by proteolytic cleavage at a specific cleavage site, for example a cleavage site for an enzyme. This may be located between the TRPC or functionally active variant thereof and the marker or tag. The cleavage site could for example be a protease cleavage site. Examples of proteases are chymotrypsin, trypsin, elastase, and plasmin; the corresponding cleavage sites are known to a person skilled in the art. Since the molecule to be purified is a protein, specific proteases, especially proteases from viruses that normally attack plants, are preferred. Examples of suitable specific proteases are thrombin, factor Xa, Igase, TEV-protease from the "Tobacco Etch Virus", the protease PreScission (Human Rhinovirus 3C Protease), enterokinase or Kex2. TEV-protease and PreScission are especially preferred.

With respect to the second alternative, the first protein of the complex of the invention is a domain of a classical transient receptor potential channel (TRPC) or a functionally active variant thereof, the domain having the sequence of SEQ ID NO:1 as defined above.

In a preferred embodiment of the invention obligatory sequence of SEQ ID NO: 1 may be further defined as follows, wherein the variable amino acids X are specified by the following indices: LX₁X₂X₃X₄X₅YQEVX₆RNLVKRYVAAMIRX₇X₈KT (SEQ ID NO:1):
- X₁X₂X₃X₄X₅ comprises preferably at least 2, preferably 3, more preferably 4 or even 5 polar (such as Asn, or Gln), optionally basic amino acids residues (such as Arg or His), especially wherein the partial sequence of X₁X₂X₃X₄X₅ may be as defined in SEQ ID NO:2 or SEQ ID NO:3;
- X₆ is preferably larger, nonpolar amino acids residue (such as Ile, Leu or Met); and/or
- X₇X₈ is preferably a combination of a acidic and a polar/neutral amino acid residue (such as Glu and Ala) or a combination of a two polar/neutral amino acid residues (such as Asn and Ser), wherein the partial sequence of X₇X₈ may be as defined in SEQ ID NO:2 or SEQ ID NO:3.

In a more preferred embodiment of the invention the sequence of SEQ ID NO: 1 may be further defined as follows:
- X₁X₂X₃X₄X₅ comprises preferably at least 2, preferably 3, more preferably 4 or even 5 polar (such as Asn or Gln ), optionally basic amino acids residues (such as Arg or His), especially wherein the partial sequence of X₁X₂X₃X₄X₅ may be as defined in SEQ ID NO:2 or SEQ ID NO:3;
- X₆ is preferably larger, nonpolar amino acids residue (such as Ile, Leu or Met); and
- X₇X₈ is preferably a combination of a acidic and a polar/neutral amino acid residue (such as Glu and Ala) or a combination of a two polar/neutral amino acid residues (such as Asn and Ser), wherein the partial sequence of X₇X₈ may be as defined in SEQ ID NO:2 or SEQ ID NO:3.

The properties of the amino acid side chains are summarized in the following:

| Amino Acid | Polarity | Acidity/Basicity |
|---|---|---|
| Alanine | nonpolar | neutral |
| Arginine | polar | basic |
| Asparagine | polar | neutral |
| Aspartic acid | polar | acidic |
| Cysteine | polar | neutral |
| Glutamic acid | polar | acidic |
| Glutamine | polar | neutral |
| Glycine | nonpolar | neutral |
| Histidine | polar | basic |
| Isoleucine | nonpolar | neutral |
| Leucine | nonpolar | neutral |
| Lysine | polar | basic |
| Methionine | nonpolar | neutral |
| Phenylalanine | nonpolar | neutral |
| Proline | nonpolar | neutral |
| Serine | polar | neutral |
| Threonine | polar | neutral |
| Tryptophan | nonpolar | neutral |
| Tyrosine | polar | neutral |
| Valine | nonpolar | neutral |

The functionally active variant of a classical transient receptor potential channel (TRPC) with respect to the second alternative of the invention is defined as that of the first alternative for the first protein.

In a preferred embodiment the functionally active variant may be defined as above, wherein the sequence of SEQ ID NO:1 is further defined in that
- it is derived from SEQ ID NO:2 or SEQ ID NO:3 by one or more amino acid substitutions, preferably at most 8, 7, 6 or 5, more preferably at most 4, still more preferably at most 3, even more preferably at most 2 and most preferably at most 1 amino acid substitution; preferably one or more of these amino acid substitutions is a conservative amino acid substitution as defined below
   or
- it is derived from any naturally occurring TRPC (see above).

Conservative substitutions are those that take place within a family of amino acids that are related in their side chains and chemical properties. Examples of such families are amino acids with basic side chains, with acidic side chains, with non-polar aliphatic side chains, with non-polar aromatic side chains, with uncharged polar side chains, with small side chains, with large side chains etc. In one embodiment, one conservative substitution is included in the peptide. In another embodiment, two conservative substitutions or less are included in the peptide. In a further embodiment, three conservative substitutions or less are included in the peptide.

Examples of conservative amino acid substitutions include, but are not limited to, those listed below:

| Original Residue | Conservative Substitutions |
|---|---|
| Ala | Ser |
| Arg | Lys |
| Asn | Gln; His |
| Asp | Glu |
| Cys | Ser |
| Gln | Asn |
| Glu | Asp |
| His | Asn; Gln |
| Ile | Leu, Val |
| Leu | Ile; Val |
| Lys | Arg; Gln; Asn |
| Met | Leu; Ile |
| Phe | Met; Leu; Tyr |
| Ser | Thr |
| Thr | Ser |
| Trp | Tyr |
| Tyr | Trp; Phe |
| Val | Ile; Leu |

In the context of the first and second alternative of the first protein, the TRPC is preferably selected from the group consisting of TRPC4, TRPC5, and TRPC6, particularly it is TRPC4 or TRPC5, especially TRPC4α or TRPC4β or TRPC5.

In another preferred embodiment of the invention the first protein comprises the sequence LRRHHQYQEVMRNLVKRYVAAMIREAKTE (SEQ ID NO:2) or LIQNQHYQEVIRNLVKRYVAAMIRNSKTN (SEQ ID NO:3).

It should be understood that in the context of the present invention, the TRPC may be derived from any species. However, mammalian TRPCs, such as those derived from human, rat, mouse, cat, dog or horse, particularly murine or human TRPCs, are preferred.

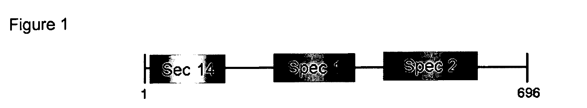

The second protein of the complex of the invention comprises or consists of the Spec 1 domain of SESTD1. The sequence of human SESTD1 is disclosed in GenBank accession number NP_835224. However natural occurring variants are also known, e.g. one was found full length in the human aorta cDNA library. Its amino acid sequence is identical to GenBank accession no. NP_835224 apart from one exchange (H508Q). It is based on a point mutation at 1571 (NM_178123) that can also be found in a genomic blast. Another point mutation found at 1748 is silent. The Spec 1 domain consists of amino acids 233 to 381 of the SESTD1 as specified above (see also Figure 1). In accordance with the present invention homologous Spec 1 sequences derived from alternative naturally occurring SESTD1 may be also used. The alternative SESTD1 may be derived from another species or organ or subject. The sequences of various SESTD1 proteins are derivable from protein databases such as NCBI (National Center for Biotechnology Information) and homologous sequences may be identified by suitable alignment programs.

In accordance with the present invention the second protein may also be a fragment of a naturally occurring SESTD1 provided that the Spec 1 domain is still present. However, the Spec 1 domain may also have added (C- and/or N-terminally) one or more amino acid sequences not related to any naturally occurring SESTD1. Preferably, if one or more amino acid sequence is added to the SESTD1, it/they is/are segments of naturally occurring SESTD1 (leading to fragments or chimeras), which may also encompass amino acids substitutions (preferably a limited number such as up to 10), preferably conservative substitutions, as defined above.

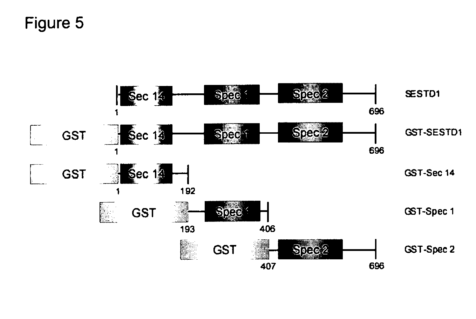

Additionally, the second protein may also encompass a marker or tag, as defined in connection with the first protein of the invention. Examples of a protein comprising SESTD1 or fragments thereof and a GST-tag are illustrated in Figure 5.

In another preferred embodiment of the invention the second protein comprises or consists of the full length SESTD1, wherein the SESTD1 may be as defined above.

It should be understood that in the context of the present invention, the SESTD1 may be derived from any species. However, mammalian SESTD1s, such as those derived from human, rat, mouse, cat, dog or horse, particularly rat, murine or human SESTD1, are preferred.

A third aspect of the invention relates to a test system comprising
- a first protein as defined above in the context of the present invention,
- a second protein as defined above in the context of the present invention and
- means for detecting interaction of the first and the second protein.

As detailed above the physiological, pathophysiological and biochemical role of the members of the TRPC family is not yet fully elucidated. Accordingly, tools for studying the function and control of TRPCs and substances modulating the same are required. The test system of the invention may be used in order to elucidate the function and activity of TRPCs. Particularly, mechanisms involving TRPC and SESTD1 can be studied by using the test system of the invention.

A series of tests are known in the art in which the test system may be used and to which the test system may be adapted. This may be a heterogeneous or homogeneous assay. As used herein, a heterogeneous assay is an assay which includes one or more washing steps, whereas in a homogeneous assay such washing steps are not necessary. The reagents and compounds are only mixed and measured.

In an embodiment the assay is an ELISA (enzyme linked immuno sorbent assay), a DELFIA (dissociation enhanced lanthanide fluoro immuno assay), an SPA (scintillation proximity assay) a flashplate assay, a FRET (fluorescence resonance energy transfer) assay, TR-FRET (time-resolved fluorescence resonance energy transfer) assay, a FP (fluorescence polarisation) assay, an ALPHA (amplified luminescent proximity homogenous assay), an EFC (enzyme fragment complementation) assay, a two hybrid assay or a coimmunoprecipitation assay.

ELISA (enzyme linked immuno sorbent assay)-based assays are offered by various companies. It is a biochemical technique used to detect the presence of an antibody or an antigen in a sample. Performing an ELISA involves at least one antibody with specificity for a particular antigen (e.g a segment of the first or second protein). In general, an unknown amount of antigen in a sample is immobilized on a surface. One then washes a particular antibody over the surface. This antibody is linked to an enzyme that produces a detecable signal such as a change in colour or fluorescene. For example, the sample with an unknown amount of antigen is immobilized on a solid support (usually a microtiter plate) either non-specifically (via adsorption to the surface) or specifically (via capture by another antibody specific to the same antigen, in a "sandwich" ELISA). After the antigen is immobilized the detection antibody is added, forming a complex with the antigen. The detection antibody can be covalently linked to an enzyme, or can itself be detected by a secondary antibody which is linked to an enzyme through bioconjugation. Between each step the plate is typically washed with a mild detergent solution to remove any proteins or antibodies that are not specifically bound. After the final wash step the plate is developed by adding an enzymatic substrate to produce a visible signal, which indicates the quantity of antigen in the sample. Older ELISAs utilize chromogenic substrates, though newer assays employ fluorogenic substrates with much higher sensitivity.

DELFIA (dissociation enhanced lanthanide fluoro immuno assay)-based assays are solid phase assay. The antibody is usually labelled with Europium or another lanthanide and the Europium fluorescence is detected after having washed away un-bound Europium-labelled antibodies.

SPA (scintillation proximity assay) and the flashplate assay usually exploit biotin/avidin interactions for capturing radiolabelled substrates. Generally the reaction mixture includes the kinase, a biotinylated peptide substrate and γ-[P³³]ATP. After the reaction, the biotinylated peptides are captured by streptavidin. In the SPA detection, streptavidin is bound on scintillant containing beads whereas in the flashplate detection, streptavidin is bound to the interior of the well of scintillant containing microplates. Once immobilized, the radiolabelled substrate is close enough to the scintillant to stimulate the emission of light.

Fluorescence resonance energy transfer (FRET) describes a radiation-free energy transfer between two chromophores. A donor chromophore in its excited state can transfer energy by a non-radiative long-range dipole-dipole coupling mechanism to an acceptor fluorophore in close proximity (typically <10 nm). As both molecules are fluorescent, the energy transfer is often referred to as "fluorescence resonance energy transfer", although the energy is not actually transferred by fluorescence. FRET is a useful tool to detect and quantify protein-protein interactions, protein-DNA interactions, and protein-conformational changes. For monitoring binding of one protein to another or one protein to DNA, one of the molecules is labeled with a donor and the other with an acceptor and these fluorophore-labeled molecules are mixed. When they are present in an unbound state, donor emission is detected upon donor excitation. Upon binding of the molecules, the donor and acceptor are brought in proximity and the acceptor emission is predominantly observed because of the intermolecular FRET from the donor to the acceptor. Suitable neighbors for FRET are known in the art and the skilled practitioner will be able to choose a suitable combination of labels for both antibodies. As used herein with respect to donor and corresponding acceptor, "corresponding" refers to an acceptor fluorescent moiety having an excitation spectrum that overlaps with the emission spectrum of the donor. However, both signals should be separable from each other. Accordingly, the wavelength maximum of the emission spectrum of the acceptor should preferably be at least 30 nm, more preferably at least 50 nm, such as at least 80 nm, at least 100 nm or at least 150 nm greater than the wavelength maximum of the excitation spectrum of the donor.

Representative donor fluorescent moieties that can be used with various acceptor fluorescent moieties in FRET technology include fluorescein, Lucifer Yellow, B-phyco-erythrin, 9-acridineisothiocyanate, Lucifer Yellow VS, 4-acetamido-4'-isothiocyanatostilbene-2,2'-disulfonic acid, 7-diethylamino-3-(4'-isothiocyanatophenyl)-4-methylcoumarin, succinimdyl 1-pyrenebutyrate, and 4-acetamido-4'-isothiocyanatostilbene-2,2'-disulfonic acid derivatives. Representative acceptor fluorescent moieties, depending upon the donor fluorescent moiety used, include LC-Red 610, LC-Red 640, LC-Red 670, LC-Red 705, Cy5, Cy5.5, Lissamine rhodamine B sulfonyl chloride, tetramethyl rhodamine isothiocyanate, rhodamine x isothiocyanate, erythrosine isothiocyanate, fluorescein, diethylenetriamine pentaacetate or other chelates of lanthanide ions (e.g., europium, or terbium). Donor and acceptor fluorescent moieties can be obtained, for example, from Molecular Probes (Junction City, OR) or Sigma Chemical Co. (St. Louis, MO).

Alternatively, time-resolved fluorescence resonance energy transfer (TR-FRET) may be used for the test system of the present invention. TR-FRET unites TRF (time-resolved fluorescence) and the FRET principle. This combination combines the low background benefits of TRF and the homogeneous assay format of FRET. While FRET has already been described above, TRF takes advantage of the unique properties of lanthanides or any other donor with long half-life. Suitable donors for TR-FRET include, amongst others, lanthanide chelates (cryptates) and some other metal ligand complexes, which can have fluorescent half-life in the micro- to millisecond time range and which, therefore, also allow the energy transfer to occur in micro- to millisecond measurements. Fluorescence lanthanide chelates have been used as energy donors in the late seventies. The commonly used lanthanides include samarium (Sm), europium (Eu), terbium (Tb) and dysprosium (Dy). Because of their specific photophysical and spectral properties, complexes of lanthanides are of major interest for fluorescence application in biology. Specifically, they have a large Stokes' shift and extremely long emission half-lives (from microseconds to milliseconds) when compared to more traditional fluorophores.

Usually, organic chromophores are used as acceptors. These include allophycocyanin (APC). Suitable details on TR-FRET as well as acceptors are described in WO 98/15830.

Fluorescence polarisation (FP)-based assays are assays which use polarized light to excite fluorescent substrate peptides in solution. These fluorescent peptides are free in solution and tumble, causing the emitted light to become depolarised. When the substrate peptide binds to a larger molecule, its tumbling rates are greatly decreased, and the emitted light remains highly polarized.

In a further embodiment of the invention, the test system of the invention is adapted for an amplified luminescence proximity homogeneous assay (ALPHA). ALPHA is a solution-based assay which was originally developed by Packard BioScience. ALPHA is a luminescence-based proximity assay, wherein one interaction partner is attached to donor beads, while the other is coupled to acceptor beads, both with a diameter of only about 250 nm. A photosensitizer compound is embedded into the donor bead. With this compound upon illumination with laser light at a wavelength of about 680 nm, ambient oxygen is converted into energy-rich, short-life singlet oxygen. When no acceptor bead is in proximity, the singlet oxygen decays without producing a signal. If donor and acceptor beads are brought together (ca. 250 nm) by the biological interaction of the attached biomolecules, the singlet oxygen released by the donor bead initiates a luminescence/fluorescence cascade in the nearby acceptor bead, leading to a highly amplified signal in the 520-620 nm range. The luminescence signal is detected in a suitable reader. For more details regarding ALPHA techniques, see Ullman et al., 1994, Proc. Natl. Acad. Sci., USA 91, 5426-5430.

EFC (enzyme fragment complementation)-based assays or equivalent assays can be used in particular for high throughput screening of compounds. The EFC assay is based on an engineered β-galactosidase enzyme that consists of two fragments - the enzyme acceptor (EA) and the enzyme donor (ED). When the fragments are separated, there is no β-galactosidase activity, but when the fragments are together they associate (complement) to form active enzyme. The EFC assay utilizes an ED-analyte conjugate in which the analyte may be recognized by a specific binding protein, such as an antibody or receptor. In the absence of the specific binding protein, the ED-analyte conjugate is capable of complementing EA to form active β-galactosidase, producing a positive luminescent signal. If the ED-analyte conjugate is bound by a specific binding protein, complementation with EA is prevented, and there is no signal. If free analyte is provided (in a sample), it will compete with the ED-analyte conjugate for binding to the specific binding protein. Free analyte will release ED-analyte conjugate for complementation with EA, producing a signal dependent upon the amount of free analyte present in the sample.

Two-hybrid screening is a molecular biology technique used to discover protein-protein interactions by testing for physical interactions (such as binding) between two proteins. The premise behind the test is the activation of downstream reporter gene(s) by the binding of a transcription factor onto an upstream activating sequence. For the purposes of two-hybrid screening, the transcription factor is split into two separate fragments, called the binding domain (BD) and activating domain (AD). The BD is the domain responsible for binding to the UAS and the AD is the domain responsible for activation of transcription. A suitable two-hybrid system is described in Example 1.

Co-immunoprecipitation can be used for the identification of protein complexes by precipitating one protein believed to be in a complex because additional members of the complex are captured as well and can be identified. The protein complexes, once bound to the specific antibody, are removed from the bulk solution by capture with an antibody-binding protein attached to a solid support such as an agarose bead. These antibody-binding proteins (Protein A, Protein G, Protein L) were initially isolated from bacteria and recognize a wide variety of antibodies. Following the initial capture of a protein or protein complex, the solid support is washed several times to remove any proteins not specifically and tightly bound through the antibody. After washing, the precipitated protein(s) are eluted and analyzed using gel electrophoresis, mass spectrometry, Western blotting, or any number of other methods for identifying constituents in the complex. Thus, co-immunoprecipitation is a standard method to assess protein-protein interaction. A suitable test system involving coimmunoprecipition is described in Example 3.

In another preferred embodiment of the invention the means for detecting the interaction between the first and second protein may be adapted to measure one or more phospholipid(s), preferably phosphatidyl-inositol-phosphate. The measuring may include the determination of the concentration of one or more phospholipid(s), preferably phosphatidyl-inositol-phosphate. The concentration may be measured in response to a potential TRPC modulator as described in the method for screening a modulator of the invention. Means and methods for determining concentrations of one or more phospholipid(s), preferably phosphatidyl-inositol-phosphate, are well known to the skilled person and include such involving radio-labelled phospholipid(s), preferably phosphatidyl-inositol-phosphate(s), including purification by e.g. column chromatography, or mass spectrometry. However, means and methods involving a PIP strip phospholipid overlay assay or a Cova-PIP plate binding assay (as exemplified in Example 8a and 8b, respectively) are preferred.

Exemplary test systems and their use are described in Example 1 to 8.

Preferably, the test system is adapted for high throughput screening. In this method a large number of compounds is screened against the TRPC in question in either cell-free or whole-cell assays. Typically, these screenings are carried out in 96 well plates using automated, robotic station based technologies or in higher-density array ("chip") formats.

In accordance with the present invention, each of the proteins of the complex of the invention may be any of the proteins as specified in the above aspects and embodiments, particularly preferred embodiments. It could be shown that TRPC is able to interact with a series of further proteins. Accordingly, the test system may further comprise at least one protein selected from the group consisting of ankyrin repeat domain 35 (ANKRD35), apolipoprotein A-I binding protein (APOA1 BP), bromodomain adjacent to zinc finger domain (BAZ1B), high-mobility group protein 2-like1 isoform b (HMG2L1), makorin RING finger protein 1 (MKRN1), pre-B-cell leukemia homeobox interacting protein 1 (PBXIP1), sarcoma antigen NY-SAR-48, spectrin alpha non-erythrocytic 1 (SPTAN1), structural maintenance of chromosomes 3 (SMC3) and talin 2 (TLN2).

The above proteins may be obtained from any species as suitable in the prevailing test system. However, exemplary proteins and their sequences are as follows:

| **Definition** | **Gene name** | **GenBank Accession No.** |
|---|---|---|
| Ankyrin repeat domain 35 | ANKRD35 | NM_144698 |
| Apolipoprotein A-I binding protein | APOA1BP | NM_144772 |
| Bromodomain adjacent to zinc finger domain | BAZ1B | AB032253 |
| High-mobility group protein 2-like1 isoform b | HMG2L1 | CR456504 |
| Makorin RING finger protein 1 | MKRN1 | NM_013446 |
| Pre-B-cell leukemia homeobox interacting protein 1 | PBXIP1 | NM_020524 |
| Sarcoma antigen NY-SAR-48 | | NM_033417 |
| Spectrin, alpha, non-erythrocytic 1 | SPTAN1 | U83867 |
| Structural maintenance of chromosomes 3 | SMC3 | AF067163 |
| Talin 2 | TLN2 | NM_015059 |

The test system may e.g. be used for the below method for screening for a TRPC modulator.

In a forth aspect the present invention relates to a method for screening for a TRPC modulator comprising the steps of:
a) contacting the test system according to the invention as specified above with a substance and
b) detecting a measurable signal upon interaction of the substance with the test system, thereby identifying the substance as a TRPC modulator.

In accordance with the present invention, the test system may be specified as detailed in the present description of the invention, particularly as detailed in the preferred embodiments. The test system may be encompassed in a cell or it may be a cell-free system. The test system may be contacted with a test substance under conditions suitable to detect a measurable signal. This includes a suitable temperature, chemical environment (buffers, pH value, etc.) as well as a suitable concentration of the substance and an appropriate time of contact.

After or simultaneously with the contacting, a signal is observed wherein the detection of a signal is indicative of the interaction of the first and the second protein.

In a preferred embodiment of the invention the influence of the substance on the interaction between the first and the second protein as defined above is detected directly, i.e. that the proximity of the first and the second protein is detected by the induction of a signal and not a signal downstream of the TRPC/SESTD1 signal transduction. The induction of a signal may be effected by labeling of each of the components, wherein the proximity of the labels induces a detectable signal. The induced detectable signal may be a chemiluminescent signal, a change in color, a fluorescence signal, a radiation or any other suitable signal. The signal may be induced by interaction of two labels, wherein each label is bound to one component of the test system, i.e. the isolated protein and the co-factor. Such signals include a radiolabel, such as ¹²⁵I, on the one hand and a suitable quencher on the other hand in order to detect proximity in a scintillation counter (scintillation proximity assay). The components may encompass antigens accessible to antibodies labeled in a manner to detect FRET (fluorescence resonance energy transfer, see above). In another alternative, one of the proteins has bound to its surface a biomolecule capable of phosphorylation by a kinase and the other component has a trivalent metal ion complexed to its surface, e.g., via a suitable linker such as nitrilotriacetic acid, iminodiacetic acid or an appropriately substituted N-containing heterocycle, for example a triazoheterocycle, for example a triazocyclononaneononane, such as 1-propylamino-4-acetato-1,4,7-triazacyclononane. A chemiluminescent signal is generated when the donor and acceptor particles are in close proximity, which occurs upon binding of the protein to the co-factor (luminescent proximity assay).

Suitable tests and assays for detection protein-protein interactions are detailed above.

In one preferred embodiment of the invention, the means for detecting the interaction between the proteins includes at least one antibody specific for the first protein or the second protein. As detailed above, the isolated protein may include an antigen for a specific antibody and the detection of the antibody. In an even more preferred embodiment of the invention, the test system comprises two antibodies, wherein the first antibody is specific for the first protein and the second antibody is specific for the second protein. The antibody may be labeled with a Suitable marker, which is indicative of the presence of the respective component. Alternatively, the above-mentioned primary antibody may be detected by a suitable secondary antibody directed against the primary antibody. The secondary antibody may be used in order to detected the presence of the primary antibody, e.g. when bound to the secondary antibody. The primary or secondary antibody may be labeled with a marker as defined above, for example, an enzyme, a radiolabel, a fluorescence marker, a chemiluminescent marker, etc. Alternatively, the proteins may encompass a tag, which is detectable with a suitable antibody.

The test system may be used in a manner that a purification system for the separation of at least one component of the complex is used and the presence of the complex or of each of the components of the complex of the proteins is detected. For example, the complex may be purified by gel electrophoresis, column chromatography, affinity purification, etc. and the complex may be detected by the presence of one signal or two signals indicative of one component of the complex or both components of the complex. For example, if a separation technique is used which is specific for one complex of the component, the detection method may be limited to the other component. Alternatively, the purification method may be not specific for one of the components, such as gel electrophoresis, and the formation of the complex may be detected by two signals, e.g. two antibodies labeled with distinguishable fluorescence markers, wherein the presence of both fluorescence markers e.g. at the same area of a gel is indicative of the complex. In accordance with this, the first and/or the second protein comprise(s) a detectable marker. Preferably, the detecting of a measurable signal involves also the use of a specific antibody against the first protein and/or a specific antibody against the second protein, as detailed above.

In a preferred embodiment of the invention the TRPC modulator enhances or preferably impairs or inhibits binding of the first protein to the second protein. A modulator of TRPC alters the activity of the TRPC in question. The alteration of activity may be activation or inhibition, thus enhancing or impairing signal transduction of the TRPC, respectively. A modulator enhancing, improving or increasing activity of the TRPC is referred to as agonist, whereas a TRPC modulator impairing, inhibiting or decreasing the activity of the TRPC is referred to as antagonist. The modulation of activity is based on specific interaction with the TRPC and not the result of unspecific interaction with the TRPC (e.g. denaturation).

In another preferred embodiment of the invention the measurable signal is one or more phospholipid(s), preferably phosphatidyl-inositol-phosphate(s). Phospholipids are a class of lipids, and a major component of all biological membranes, along with glycolipids, Cholesterol and proteins. Phosphatidyl-inositol is the substrate for a large number of enzymes which are involved in cell signaling because it can be phosphorylated by a variety of kinases on the hydroxyl groups 3, 4 and 5 on the inositol ring in seven different combinations. The concentration of phospholipid(s), preferably phosphatidyl-inositol-phosphate(s), may be determined as described above.

In another embodiment of the screening methods of the invention the test compound (substance) is provided in the form of a chemical compound library. Chemical compound libraries include are plurality of chemical compounds and have been assembled from any of multiple sources, including chemically synthesized molecules and natural products, or have been generated by combinatorial chemistry techniques. They are especially suitable for high throughput screening. They may be comprised of chemical compounds of a particular structure or compounds of a particular creature such as a plant. In the context with the present invention the chemical compound library is preferably a library comprising proteins and polypeptides or small molecules.

In accordance with the above functions of TRPC, methods of the invention may be used for screening for a medicament for preventing and/or treating a disease involving endothelial dysfunction.

Particularly, the method may be used for screening for a medicament for preventing and/or treating a cardiovascular disease, cardiac hypertrophy, heart failure, ischemia, neointima hyperplasia, idiopathic dilated cardiomyopathy, hypertension, coronary syndrome, heart failure, renal failure, thrombosis; asthma, a chronic respiratory disease, chronic obstructive pulmonary disorder, idiopathic pulmonary hypertension, acute hypoxic pulmonary vasoconstriction, inflammatory occlusive disease and atherosclerosis.

In a further aspect the invention relates to the use of a test system according to the invention as specified above for the identification of a TRPC modulator, wherein the above details on the test system of the invention and the screening method of the invention are to be applied analogously.

In the following, the present invention is illustrated by examples and figures which are not intended to limit the scope of the present invention.

### EXAMPLES

### Example 1: Yeast two-hybrid (Y2H) screen with TRPC as bait

Endothelial dysfunction is believed to be the major cause of various cardiovascular diseases. Among others TRPC4 plays a critical role in endothelial function, it is associated with regulation of endothelium-dependent vascular relaxation, may contribute to oxidative-stress induced endothelial damage and is involved in endothelial barrier function.

The MATCHMAKER two-hybrid system 3 (Clontech, Mountain View, USA) was to screen for novel proteins that interact with the cytosolic C-terminus of mTRPC4α (aa 615-974) in yeast strain AH109. The system is based on the binding of the transcription factor GAL4 upstream of four reporter genes (ADE2, HIS3, IacZ, MEL1) activating their translation and protein expression. DNA binding and activation are mediated by two different protein domains which can be physically separated (Fields S & Song O (1989). A novel genetic system to detect protein-protein interactions. Nature 340, 245-246). The C-terminus of mTRPC4α (aa 615-974) served as bait. So far, no X-ray structures of TRPC channels exist but due to similarity with voltage-gated K⁺ channels, they are expected to form tetramers. We wanted to make sure that the protein assembled in the physiological multimeric state. Therefore, the bait was constructed by fusing the C-terminus of murine TRPC4α (NM_016984) with a leucine zipper domain that has been shown to direct protein tetramerization (Harbury PB, Zhang T, Kim PS, & Alber T (1993). A switch between two-, three-, and four-stranded coiled coils in GCN4 leucine zipper mutants. Science 262, 1401-1407). The amino acid sequence of the zipper (underlined) and flanking sequences is GGGSG SRMKQ IEDKL EEILS KLYHI ENELA RIKKL LGERG GSGS AAA (SEQ ID NO: 4). This construct was finally integrated into pGBKT7 (Clontech, Mountain View, USA), leading to its fusion with the GAL4-DNA binding domain (mTRPC4α (615-974)/leucine zipper/pGBKT7). Other baits for directed yeast two-hybrid screens were constructed the same way with the C-termini of human TRPC1 (NM_003304), murine TRPC5 (NM_009428) and human TRPC6 (NM_004621). For control experiments the channel C-termini were replaced by the Enhanced Green Fluorescent Protein (EGFP/leucine zipper/pGBKT7).
A human aorta cDNA library (in pACT2, Clontech, Mountain View, USA) expressed as a fusion to the GAL4 activation domain (AD) was cotransfected with the bait into yeast. Only interaction of the bait with a library protein brought DNA-BD and AD into sufficient spatial proximity reconstituting the functional GAL4 and thus leading to the expression of the reporter genes. These allowed survival of the auxotroph yeast strain AH109 on selective media lacking the otherwise essential adenine (Ade) and histidine (His). Moreover, GAL4 activity led to the expression of β-galactosidase that could be detected in yeast colonies by conversion of X-gal into a blue product. Prey plasmids from such colonies were isolated and analytically digested with the restriction endonucleases EcoRI/XhoI that cut the cDNA inserts out of the vector multiple cloning site. The resulting fragments were separated by gel electrophoresis whereby fragments of identical size pointed towards identical preys. Subsequently, they were tested for intrinsic DNA-binding and/or transcriptional activity by cotransformation with the unrelated enhanced green fluorescent protein from *Aequorea victoria* that was also tetramerized and fused to the DNA-BD (EGFP/leucine zipper/pGBKT7). Yeast cotransformed with preys that did not interact unspecifically with EGFP did not survive on -Trp/-Leu/-Ade/-His plates. In parallel, prey plasmids had been cotransformed with the bait and were retested for growth on -Trp/-Leu/-His/-Ade agar plates. Prey plasmids from surviving clones were finally sequenced and the sequences were compared with Genbank data by using BLAST software.
The human aorta MATCHMAKER cDNA library (Clontech, Mountain View, USA) was provided transformed into *E*. *coli* BNN132 and was amplified to gain enough plasmid for screening in yeast. First its titer was determined by plating dilutions of 1x 10⁻⁶, 1x 10⁻⁷, and 2x 10⁻⁷ on LB/amp agar plates. After they were incubated for 2 days (30 °C), the number of grown colonies was counted and resulted in 1.8x 10⁸ colony forming units (cfu) per mL. To obtain at least 2 - 3x the number of independent clones present in the library before amplification (3.5x 10⁶ as provided by the manufacturer) 45,000 cfu/ 150-mm plate were spread on 160 LB/amp agar plates (1.5% Bacto agar, 1% Bacto tryptone, 0.5% Bacto yeast extract, 1% NaCl, 100 µg/mL ampicilline). After 36 hrs incubation at 30 °C, cells were scraped into liquid LB/amp, pooled, partitioned into 4 aliquotes, and pelleted by centrifugation. Bacteria were lysed and plasmids purified using the QlAfilter Plasmid Giga Kit (Qiagen, Hilden, Germany).

For transformation of yeast the DNA-BD/bait and the library were sequentially polyethylenglycol/lithium acetate (PEG/LiAc)-mediated transformed into yeast (Ito H, Fukuda Y, Murata K, & Kimura A (1983). Transformation of intact yeast cells treated with alkali cations. J Bacteriol 153, 163-168).
Through a small-scale transformation, the DNA-BD/bait was introduced into yeast. An overnight culture of *Saccharomyces cerevisae* AH109 grown at 30°C in liquid YPAD medium (30 mg/L adenine, 0.65g/L complete supplement mixture, 6.7g Difco yeast nitrogen base w/o amino acids, 2% (w/v) glucose) was transferred to 10 mL YPAD medium resulting in an A₆₀₀=0.1. The cells suspension had been grown at 30°C until the logarithmic growth phase was reached (A₆₀₀=0.5), washed with sterile deionized water and resuspended in 200 µL lithium acetate buffer (100 mM LiAc, 10 mM Tris-HCl, 1 mM EDTA). 1.5 µg DNA-BD/bait (3 µg plasmids for coexpressions) were mixed with 18 µL heat-denaturated carrier DNA (from salmon sperm, Sigma, Munich, Germany), boiled for 40 seconds and transferred to ice. To 200 µL of the yeast suspension 1.2 mL PEG (40% PEG, 100 mM LiAc, 10 mM Tris-HCl, 1 mM EDTA) were added and incubated for 30 min at 30°C. Cells were heat-shocked at 42°C for 15 min, centrifugated, resuspended in 100 µL TE-Buffer, spread on selective media, and incubated at 30°C for at least 2 days. Yeast transformed with the DNA-BD/bait grew on -Trp medium, cotransformants with a prey grew on -Trp/-Leu medium. It was verified that the bait is not intrinsic DNA-binding and/or transcriptional active by cotransformation with the negative control vector pGADT7. pGADT7 is an empty vector leading to sole expression of the GAL4-AD in yeast. Since this plasmid does not express a TRPC4-interacting protein, cotransformation with the bait is not sufficient to reconstitute GAL4, thus yeast did not survive on -Trp/-Leu/-Ade/-His plates.
Single DNA-BD/bait transformants were used as inoculates for the following large-scale transformation with the AD fusion library. Three overnight cultures were transferred to 150 mL -Trp medium, divided into 12 parts in 50 mL Falcon tubes and incubated overnight at 30 °C. Next day these starter cultures were pooled, pelleted by centrifugation (3 min, 2,000 x g, RT) and resuspended in 10 mL -Trp medium. This suspension was added to 900 mL -Trp medium (A6₀₀=0.15-0.25) and cells grew until the logarithmic growth phase was reached (A₆₀₀=0.45-0.75). The suspension was subdivided onto several 50 mL Falcon tubes, pelleted by centrifugation (5 min, RT, 2,000 x g), washed with 300 mL sterile deionized water and reunited in 10 mL sterile deionized water. After washing with 50 mL lithium acetate buffer (100 mM LiAc, 10 mM Tris-HCl, 1 mM EDTA), cells were resuspended in 4 mL lithium acetate buffer. In triplicates, 40 µg cDNA of the library were mixed with 145 µL heat denaturated carrier DNA (from salmon sperm), 1.2 mL cell suspension and 8.6 mL PEG (40% PEG, 100 mM LiAc, 10 mM Tris-HCl, 1mM EDTA) and incubated at 30°C for half an hr at 250 rpm. After addition of 1 mL DMSO per tube, cells were heat shocked 15 min at 42°C, then cooled down on ice and pelleted by centrifugation (5 min, 2,000 x g, RT). The pellets were each resuspended in 25 mL -Trp/-Leu medium, reunited and grown at 250 rpm and 30 °C for one hr. After centrifugation (5 min, 2,000 g, RT), the cells were resuspended in 12 mL -Trp/-Leu medium, plated in 300 µL aliquots on -Trp/-Leu/-His agar plates (150 mm diameter), and incubated at 30°C for 2 to 5 days. Grown colonies were transferred to -Trp/- Leu/-His/-Ade agar plates and incubated for 2 to 3 more days. Surviving colonies were tested with the β-galactosidase assay and plasmids of positive reacting clones were isolated and sequenced. To test the transformation efficiency, dilutions of 10⁻³ to 10⁻⁶ were plated on -Trp/-His agar plates and the number of cfu was counted after incubating the plates for 2 days at 30°C.

For the β-galactosidase assay clones grown on Trp/-Leu/-His/-Ade agar plates were top-layered with X-gal agar (0.5 % agarose, 500 mM NaPO₄ buffer (pH 7), 1 % sodium dodecyl sulphate (SDS), 2 % X-gal in dimethylformamide (DMF)) and incubated at 30°C up to 12 hrs. Generation of a blue product was visually checked.

For plasmid preparation from yeast, clones surviving on -Trp/-Leu/-His/-Ade agar plates and positive reacting in the β-galactosidase assay were resuspended in 2 mL -Leu medium and grown 24 hrs at 30°C and 250 rpm. They were pelleted by centrifugation, and resuspended in 200 µL lysis buffer (2% Triton X-100, 1 % SDS, 100 mM NaCl, 10 mM Tris-HCl (pH 8), 1mM EDTA). 200 µL phenole/chloroforme/isoamyl alcohol (25:24:1) and 100 µL acid-treated glass beads were added and the cell walls were broken by vortexing this mixture. After centrifugation (5 min, 10,000 x g, RT) 135 µL of the aqueous phase were transferred to a new tube, mixed with 15 µL sodium acetate solution (10 %) and 375 µL ethanol, and the DNA was precipitated by centrifugation (30 min, 10,000 x g, RT). The DNA pellet was washed with ethanol (75%), dried at 37°C for 30 min, and solved in 10 µL Tris-HCl (10 mM, pH 8.5). 2 µL DNA were transformed by electroporation into *E. coli*, bacteria were spread on LB kanamycine agar plates and the plasmids were amplified and purified using the QlAprep Spin Miniprep Kit (Qiagen, Hilden, Germany).

DNA cycle sequencing reactions were performed based on the dideoxy terminator method (Sanger *et al*., 1977) with four differentially fluorescent labelled dideoxynucleotides (Parker *et al*., 1996). PCR fragments, generated by using the ABI PRISM BigDye terminator cycle sequencing ready reaction kit, were electrophoretically separated, detected, and analyzed on an ABI PRISM 3100 genetic analyzer.

Eleven proteins (listed in following table) were found to physically interact with the mTRPC4α C-terminus in the Y2H assay.

**Table 1: YTH preys.**

| **Definition** | **Gene name** | **Gene Bank Accession No.** |
|---|---|---|
| Ankyrin repeat domain 35 | ANKRD35 | NM_144698 |
| Apolipoprotein A-I binding protein | APOA1BP | NM_144772 |
| Bromodomain adjacent to zinc finger domain | BAZ1B | AB032253 |
| High-mobility group protein 2-like1 isoform b | HMG2L1 | CR456504 |
| Makorin RING finger protein 1 | MKRN1 | AM_013446 |
| Pre-B-cell leukemia homeobox interacting protein 1 | PBXIP1 | NM_020524 |
| Sarcoma antigen NY-SAR-48 | | NM_033417 |
| SEC14 and spectrin domains 1 | SESTD1 | NM_178123 |
| Spectrin, alpha, non-erythrocytic 1 | SPTAN1 | U83867 |
| Structural maintenance of chromosomes 3 | SMC3 | AF067163 |
| Talin 2 | TLN2 | NM_015059 |

None of these proteins has been described before to interact with the TRPC4 channel. SESTD1 seemed to be the most interesting potential interaction partner because a domain search (Marchler-Bauer A & Bryant SH (2004). CD-Search: protein domain annotations on the fly. Nucleic Acids Res 32, W327-W331) revealed an N-terminal SEC14p-like lipid-binding domain in SESTD1 **(****Figure 1****).** The conserved SEC14-motif is known to bind and transport cellular phospholipids.

Several reports have shown regulation of TRP channels by phospholipids, in particular PIP₂. This let us to assume that SESTD1 is involved in the regulation of TRPC4. In addition to the SEC14p-like lipid-binding domain, two helical structures called spectrin repeats were found in SESTD1. These domains are known to mediate protein-protein interactions and are found in several cytoskeletal proteins.

SESTD1 was found full length in the human aorta cDNA library. Its amino acid sequence is identical to GenBank accession no. NP_835224 apart from one exchange (H508Q). It is based on a point mutation at bp 1571 (NM_178123) that can also be found in a genomic blast. Another point mutation found at bp 1748 is silent.

### Example 2: Interaction site mapping from mTRPC4α side

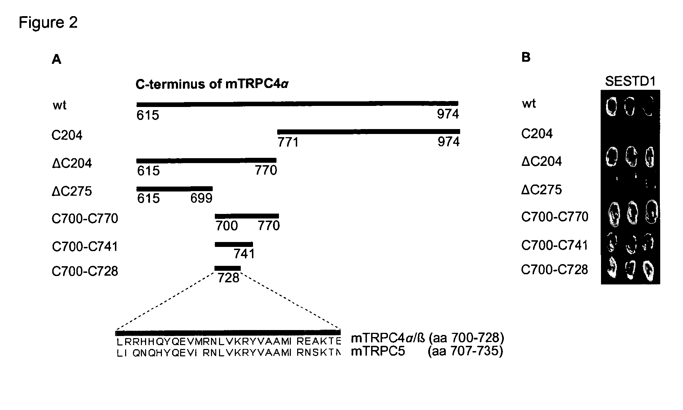

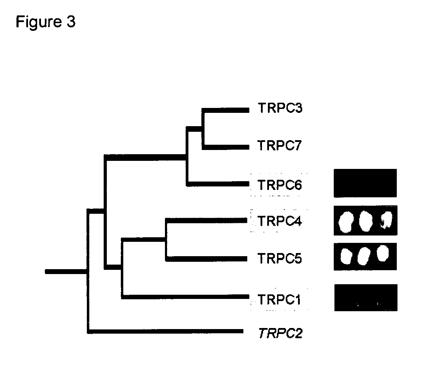

In order to define the interaction site of TRPC4 with SESTD1 in more detail a directed yeast two-hybrid screen (see Example 1) was performed. Yeast was cotransformed with SESTD1 and various C-terminal mTRPC4α fragments and plated on selective media. Experiments were performed in analogy to those in Example 1. However, for mapping of the SESTD1 interaction site on TRPC4, monomeric bait constructs were generated (lacking the zipper) to facilitate cloning.
By iterative shortening of TRPC4 fragments we found that a small sequence of 29 amino acids (aa 700-728) was sufficient to mediate the interaction with full length SESTD1 (Figure 2). This putative SESTD1 binding sequence is conserved in the shorter TRPC4β isoform as well as in TRPC5.
To verify the specificity of the interaction between SESTD1 and TRPC4/5 yeast was cotransformed with SESTD1 and the C-terminus of hTRPC1, mTRPC4β, mTRPC5 or hTRPC6 and plated on selective media. The results indicated a specific interaction of SESTD1 with the C-terminal tails of TRPC4 and TRPC5 (Figure 3).

### Example 3: GST pulldown

In order to confirm the physical interaction between SESTD1 and mTRPC4 by a protein biochemical method GST pulldown assays were performed.
A construct for the recombinant expression of a Glutathione S-transferase (GST) fusion protein in bacteria was prepared by inserting human SESTD1 (NM_178123) into the pGEX-4T-1 vector (Amersham, Munich, Germany) thus leading to its N-terminal fusion with GST.

GST fusion protein expression from pGEX vectors is under control of the tac promoter and inducible by the lactose analog isopropyl β-D thiogalactoside (IPTG). BL21 star (DE3) one shot chemically competent *E. coli* (Invitrogen, Karlsruhe, Germany) transformed with the respective plasmid were cultivated overnight in selective LB medium (containing 0.2% (m/v) glucose to repress protein expression) and subsequently transferred into 100 mL selective LB medium (with 0.2% (m/v) glucose) resulting in A₆₀₀=0.1. This liquid culture was grown at room temperature and 250 rpm to A₆₀₀=0.6-0.8. Protein expression was induced by adding 10 mM (GST) or 20 mM (GST-SESTD1, GST-SESTD1-fragments) IPTG and incubation was continued for 6 hrs. By centrifugation (16,000 x g, 1 min, RT) the bacteria suspension was pelleted in 5 mL portions, which were washed once with 2 mL D-PBS (w/o Ca²⁺, Mg²⁺) and stored at -80°C or lysed immediately. Each pellet was resuspended in 250 µL lysis buffer (containing 1 mg/mL lysozyme) and incubated on ice for 20 min. After sonication (1x 5 sec) the lysate was centrifugated 30 min at 16,000 x *g* and 4°C and the supernatant was transferred to a fresh vial. 50 µL Glutathione sepharose suspensions were washed with 500 µL lysis buffer (using a G27 needle) three times before the supernatant was added and filled up to 1 mL with lysis buffer. After incubation for 1 hr on a rotator (room temperature) the beads were washed three times with 500 µL lysis buffer. GST fusion proteins bound to glutathione sepharose were used for the GST pulldown assays.

HEK293 cells transiently transfected with the mTRPC4α-C-terminus or full length mTRPC4α were lysed and protein concentration determined using the BCA protein assay kit. An equivalent of 50 µg total protein was added to GST or GST-SESTD1 bound to glutathione sepharose, filled up to 1 mL with lysis buffer and incubated on a rotator (2 hrs, RT). Sepharose was washed three times with 500 µL lysis buffer and a G27 needle before 20 µL 1x LDS sample buffer (containing 5 % β-ME) (Invitrogen, Karlsruhe, Germany) were added. Probes were denaturated (95 °C, 3 min), centrifugated and analyzed by Western blot. For Western blotting, samples were loaded on Bis-Tris-HCl gradient gels (4-12%) and electrophoretically separated in NuPAGE MOPS SDS running buffer for 75 min at 150 V. Gels were blotted onto a nitrocellulose membrane using NuPAGE Transfer Buffer (Invitrogen, Karlsruhe, Germany) and a Novex XCell 2 Blotmodul (Invitrogen, Karlsruhe, Germany) or the iBlot Dry System (Invitrogen, Karlsruhe, Germany) according to the manufacturer's instruction. To verify transfer, the proteins on the nitrocellulose membrane were stained with 0.1% Ponceau S Solution (Sigma, Munich, Germany) for 1 min and excessive dye was removed by washing with water. The membrane was blocked with blocking buffer (Odyssey blocking buffer diluted 1:1 with 0.6% Tween 20 containing TBS (500 mM NaCl, 20 mM Tris-HCl, pH 7.4) for one hr (RT) before incubating it with the primary antibody (1 hr, RT) (see below list of antibodies). After washing it four times with TBST (500 mM NaCl, 20 mM Tris-HCl, 0.05% Tween 20, pH 7.4) it was incubated with the fluorescent labelled antibody (45 min, RT) (see list of antibodies) and washed again four times with TBST. Membranes were analyzed using the Odyssey Infrared Imaging System (LiCor, Lincoln, USA) or the Lumi Imager (Roche, Mannheim, Germany).

**Table 2: List of used antibodies (for this and the following examples).**

| | |
|---|---|
| Alexa Fluor 680 anti-mouse (rabbit) | Invitrogen, Karlsruhe, Germany |
| Alexa Fluor 680 anti-rabbit (goat) | Invitrogen, Karlsruhe, Germany |
| Alexa Fluor 680 anti-rat (goat | Invitrogen, Karlsruhe, Germany |
| anti-FLAG (rabbit) | Rockland, Gilbertsville, USA |
| anti-GAPDH (mouse) | Chemicon, Wiesbaden, Germany |
| anti-GFP (mouse) | Roche, Mannheim, Germany |
| anti-GST (rabbit) | Sigma, Munich, Germany |
| anti-GST (rabbit), Eu-N1 labelled | Perkin Elmer, Waltham, USA |
| anti-HA (rat) | Roche, Mannheim, Germany |
| anti-rabbit (goat), Eu-N1 labelled | Perkin Elmer, Waltham, USA |
| anti-rabbit (goat), horseradish peroxidase | Pierce, Rockford, USA |
| (HRP)-conjugated | |
| anti-CECTD1 #147/148 (rabbit), 0.18 mg/mL | Eurogentec, Seraing, Belgium |
| anti-TRPC4 (rabbit) | Alomone, Jerusalem, Israel |
| anti-TRPC5 (rabbit) | Alomone, Jerusalem, Israel |

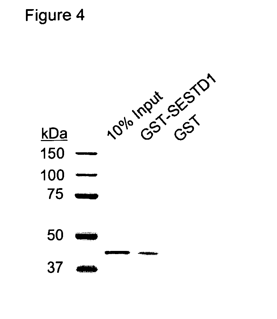

GST pulldown assays were performed to confirm the physical interaction between SESTD1 and mTRPC4 by a protein biochemical method. In *E. coli* expressed and purified recombinant GST-SESTD1 was first used to pull down overexpressed full length mTRPC4α from a HEK293 cell lysate. However, as both proteins have the same size the channel signal detected by an anti-TRPC4 antibody could be feigned by unspecific binding to SESTD1. To circumvent this problem, the smaller mTRPC4α C-terminus (aa 615-974) was used in further pulldown experiments with GST-SESTD1. In these experiments it could be demonstrated that SESTD1 and the mTRPC4α-C-terminus can physically interact **(****Figure 4****).**

### Example 4: Interaction site mapping from SESTD1 side

The GST pulldown approach (see Example 3) was adapted and used to investigate the interaction site between mTRPC4α and SESTD1. According to the predicted domains three SESTD1-constructs (see Figure 5) were cloned into pGEX-5X-3 (Amersham, Munich, Germany) and expressed as GST fusion proteins in One shot BL21 star (DE3) chemically competent *E. coli* (Invitrogen, Karlsruhe, Germany).

Under the chosen conditions it was not possible to purify desired amounts of GST-Sec 14 (aa 1-192) with the SEC14p-like lipid-binding domain from bacteria. Induction of its expression seemed to be toxic for bacteria as they grew much slower than bacteria transformed with GST-Spec 1 (aa 193-406), GST-Spec 2 (aa 407-696) or full length GST-SESTD1.

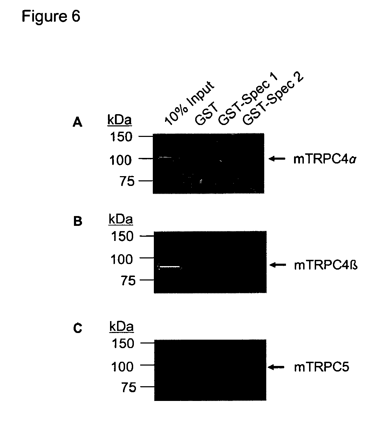

As the mapped SESTD1-binding site in mTRPC4α is also present in the TRPC4-β isoform and is highly conserved in mTRPC5 we tested for interaction of all three channels with the GST-linked SESTD1 fragments. mTRPC4α -β and mTRPC5 all strongly interacted with the first spectrin domain and in some blots a weak binding to the second domain could also be observed. The first spectrin domain seems to be the main site of interaction but one cannot exclude participation of the SEC14p-like lipid-binding domain **(****Figure 6****).**

Because we were unable to perform pulldown experiments with the SEC 14 domain, a directed yeast two-hybrid screen was employed to define in yet more detail the involvement of the different SESTD1 domains in TRPC4/5 binding. Three SESTD1 constructs, SESTD1-Sec 14 (aa 1-192), SESTD1-Spec 1 (aa 193-406) and SESTD1-Spec 2 (aa 407-696) were cloned analogously to the depicted constructs in **Figure 5** by inserting the respective SESTD1 gene fragments into the yeast expression vector pACT2 (Clontech, Mountain View, USA) leading to their expression as a fusion to the GAL4 activation domain.

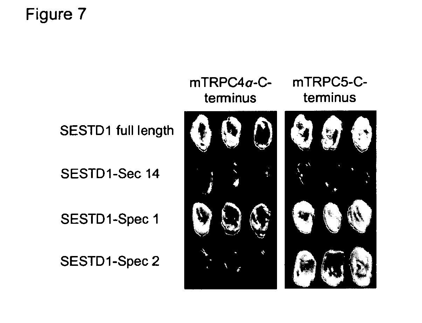

The experimental results confirmed the first spectrin domain as site of interaction between SESTD1 and the mTRPC4α C-terminus. It also showed that the SEC14p-like lipid-binding domain is not involved **(****Figure 7****).** The weak interaction between the Spec 2 domain and the mTRPC4α C-terminus observed in some GST pulldown experiments was not strong enough to allow survival of cotransformed yeast on selective media. In contrast the mTRPC5-C-terminus independently interacted with the Spec 1 and the Spec 2 domain in the directed yeast two-hybrid. In summary the results from the adapted GST pulldown and the directed yeast two-hybrid assay identify the Spec 1 domain as the main interaction side in SESTD1.

### Example 5: Coimmunoprecipitation

The previous Y2H and GST pulldown experiments had shown that SESTD1 interacts with mTRPC4□, -β and mTRPC5. Coimmunoprecipitation experiments were performed to verify that these protein-protein interactions also occur *in vivo.* As there are no commercial antibodies against SESTD1 available, two polyclonal peptide antibodies were custom-made by Eurogentec (Seraing, Belgium) and characterized. Anti-SESTD1 #147 antibody was directed against a sequence within the first spectrin domain (aa 265-280, CRQRSKRTQLEEIQQK; SEQ ID NO:5) and anti-SESTD1 #148 was generated by immunizing rabbits with the C-terminus (aa 682-696, KRQQLRHPEMVTTES; SEQ ID NO:6). Antibodies were supplied affinity-purified on the respective peptides.

hSESTD1 was inserted into the vector pCMV-HA (Invitrogen, Karlsruhe, Germany) leading to its expression as a N-terminal fusion protein with the hemagglutinin antigen epitope of human influenza virus (HA tag: YPYDV PDYA; SEQ ID NO: 7). HM1 cells (a HEK293 cell line stably expressing the muscarinic M1 receptor (Peralta EG, Ashkenazi A, Winslow JW, Ramachandran J, & Capon DJ (1988). Nature 334, 434-437)) transiently transfected with HA-SESTD1 as well as non-transfected HM1 cells were lysed (1 mM EDTA, 150 mM NaCl, 50 mM Tris-HCl, 1% Triton X-100, supplemented with Complete protease inhibitor) and the protein concentration was determined using the BCA protein assay kit..

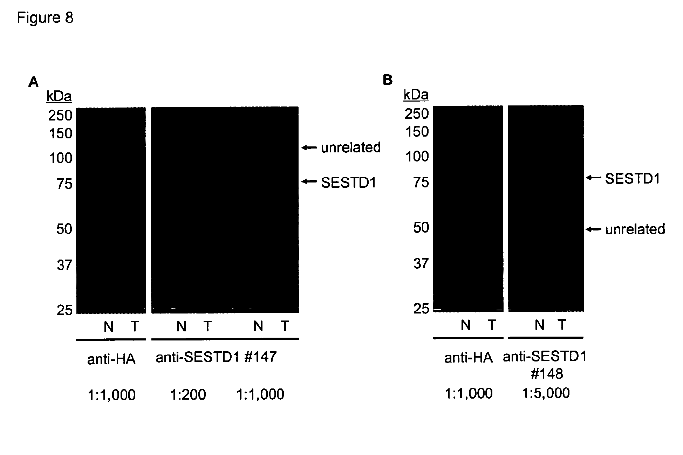

For Western blotting, equal amounts of protein samples were loaded on Bis-Tris-HCl gradient gels (4-12%) and electrophoretically separated (see Example 3). When tested in Western blot, both antibodies detected overexpressed HA-tagged SESTD1 in HM1 cell lysates (expected size 79 kDa). In addition, in nontransfected cells an endogenous protein co-migrating with HA-SESTD1 was recognized by both antibodies strongly suggesting that this protein is native SESTD1. In addition to SESTD1, anti-SESTD1 #147 recognized several other proteins on Western blots independently of the dilution used and anti-SESTD1 #148 also detected another protein with an apparent mass of 50 kDa **(****Figure 8****).**

HM1 cells were cotransfected with HA-tagged SESTD1 and FLAG-tagged mTRPC4β, GFP-tagged mTRPC5 or pcDNA3.1 (negative control) respectively. Cells were harvested by placing them on ice for 10 min before washing them twice with 10 mL ice-cold D-PBS (w/o Ca²⁺, Mg²⁺) 24 hrs after transfection. Cell membranes were isolated by scraping cells from one dish into 600 µl homogenization buffer (320 mM sucrose, 5 mM HEPES, pH 7.4, supplemented with Complete protease inhibitor). The cell suspensions from four dishes per transfection condition were united, sonicated (1 x 5 sec), centrifugated (100 x g, 10 min) and the resulting supernatant was further centrifugated partitioned onto three vials (100,000 x g, 30 min). Membrane proteins were lysed from the resulting pellets with 600 µL lysis buffer (supplemented with Complete protease inhibitor) per pellet, incubated on ice for 1 hr and centrifugated (15 min, 16000 x g, 4°C). The supernatants were transferred to a fresh vial and united. An aliquot was kept as input control and the rest of the lysate was divided onto two vials. To each probe 4 µg of the first antibody were added and they were incubated overnight at 4 °C on a rotator. Cell lysates were gained from four dishes per transfection condition. Cells from one dish were directly scrapped into 600 µL lysis buffer (supplemented with Complete protease inhibitor), incubated on ice for 1 hr and centrifugated (15 min, 16000 x g, 4°C). The supernatant was transferred to a fresh vial, united with the lysates of the 3 remaining dishes and treated as described above. Next day 30 µL protein A-sepharose or protein G-sepharose suspension, respectively, were washed and added to the probes. They were incubated 2 more hrs at 4°C. Sepharose was washed three times with a gauge 27 needle and 500 µL lysis buffer before 20 µL 2x LDS sample buffer (containing 10% β-ME) were added. Probes were denaturated (95°C, 3 min), centrifugated and analyzed by Western blot (see Example 2) with anti-SESTD1 antibody. Successful precipitation of the ion channels was checked by probing with anti-FLAG (mTRPC4β) and anti-TRPC5.

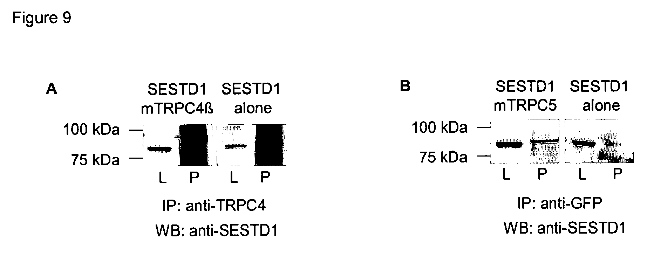

When mTRPC4β and mTRPC5 were precipitated from HM1 cell lysates, SESTD1 was found in the precipitated samples. A very small amount of SESTD1 was also precipitated by FLAG and GFP antibodies from control HM1 cell lysates that only expressed HA-SESTD1 but no FLAG-tagged mTRPC4β or GFP-tagged mTRPC5. This unspecific binding was seen under different precipitating condition. It was always much lower than coimmunoprecipitation with the ion channel proteins (Figure 9).

### Example 6: Functional interaction of SESTD1 and TRPC5

For functional interaction studies of TRPC5 and SESTD1 a HM1 cell line stably expressing mTRPC5-YFP (HM1-C5Y cells) was generated.

HM1 cells were transfected with 4 µg mTRPC5-YFP construct using Lipofectamine 2000. The construct was generated by inserting a Xbal/Notl fragment of mTRPC5-GFP (Strubing C, Krapivinsky G, Krapivinsky L, & Clapham DE (2003). Formation of novel TRPC channels by complex subunit interactions in embryonic brain. J Biol Chem 278, 39014-39019) into pcDNA3.1(-)zeo. The YFP fusion was obtained by joining a Notl/EcoRl cut YFP-PCR fragment to the channel C-terminus. 24 hrs post transfection clonal selection started by cultivating the cells in complete medium (DMEM/Nutrient F12 (with glutaMAX I; Invitrogen, Karlsruhe, Germany) supplemented with 10% (v/v) FBS (PAA, Pasching, Austria), 1 mM glutamine 400 µg/mL geneticine, 50 µg/mL zeocin (all from Invitrogen, Karlsruhe, Germany)). After several days selection single clones were isolated by manual picking of grown colonies. One clone was selected for further studies. Functional expression of the ion channel was tested by electrophysiological measurements. Current densities of carbachol and trypsin-induced ion currents measured as described previously (Strubing C, Krapivinsky G, Krapivinsky L, & Clapham DE (2001). TRPC1 and TRPC5 form a novel cation channel in mammalian brain. Neuron 29, 645-655) were 15.9 ± 5.5 pA/pF (n = 10, carbachol) and 127.8 ± 57.6 pA/pF (n = 6, trypsin). In contrast, no significant currents were induced by carbachol or trypsin in non-transfected HM1 cells.

Changes in cytosolic calcium [Ca²⁺]_{¡} were measured using the Ca²⁺-sensitive fluorescent dye fura-2 AM (Invitrogen, Karlsruhe, Germany), a widely used indicator whose fluorescent excitation maximum, upon Ca²⁺ binding, shifts towards shorter wavelengths while the fluorescence emission maximum is relatively unchanged. Typically the ratio of the fluorescence intensities excited at 340 nm and 380 nm is measured.

20,000 - 40,000 cells/well were seeded in black poly-L-lysine coated glass bottom 96-well plates (Greiner, Frickenhausen, Germany) and grew overnight to an almost confluent monolayer. They were loaded in 100 µL standard extracellular solution (E) composed of 135 mM NaCl, 1 mM MgCl₂, 5.4 mM KCl, 2 mM CaCl_{2,},10 mM HEPES, 10 mM glucose (pH 7.35) and supplemented with 2 µM fura-2 AM (30 min, 37°C) and allowed to de-esterify four 15 min at 37°C in 80 µL E. Intracellular calcium signals were ratiometrically measured in a benchtop scanning fluorometer (FLEX; Molecular Devices, Sunnyvale, CA, USA). Fluorescence was excited alternating at 340 nm and 380 nm, long-pass filtered at 495 nm and captured at 4 sec intervals The 340/380 nm excitation ratio was calculated using the SoftMax Pro software (Molecular Devices). Baseline fluorescence was detected for 30 sec before the muscarinic agonist carbachol or the protease-activated receptor (PARs) agonist trypsin was applied with the FLEX pipettor. Both carbachol and trypsin couple, via M1-receptors and PARs respectively, to phospholipase C activation in HM1 cells. Phospholipase C in turn stimulates Ca²⁺ release from intracellular stores (PI response) as well as activation of TRPC5. Ca²⁺ release from stores and Ca²⁺ entry through TRPC5 is measured fluorometrically in fura-2-loaded cells. When extracellular Ca²⁺ is absent, the carbachol- and trypsin-induced rise in intracellular Ca²⁺ is solely due to release from internal stores and independent of TRPC5. Thus, Ca²⁺ release was calculated as the area under the fluorescence curve measured in Ca²⁺ free extracellular solution. TRPC5-mediated Ca²⁺ influx was calculated by subtracting Ca²⁺ release from the area under the curve measured in standard (Ca²⁺ containing) extracellular solution.

When HA-SESTD1 was overexpressed in HM1-CY5 cells, TRPC5-mediated Ca²⁺ influx following application of carbachol or trypsin did not significantly differ from control cells transfected with an unrelated protein (β-galactosidase, bGAL). Ca²⁺ releases from internal stores were also not significantly changed in the presence or absence of HA-tagged SESTD1.

Overexpression of HA-SESTD1 in a HM1 cell line that endogenously expresses SESTD1 (Example 5) did not influence TRPC5-mediated Ca²⁺ influx. Therefore, it was tested whether knock-down of SESTD1 protein expression in these cells modified TRPC5 function.

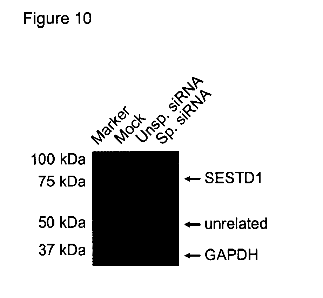

Knock-down of SESTD1 was accomplished by transfection of cells with a pool of three siRNA duplexes directed against different SESTD1 sequences (Dharmacon, sequences see Table 2). Since FBS had no significant effect on transfection efficiency, cells were transfected in full medium using Lipofectamine 2000 (Invitrogen, Karlsruhe, Germany). Only the liposomes were formed and loaded in transfection medium (Opti-MEM, also from Invitrogen). 3x10⁵ HM1 cells/2mL/well were seeded in a 6-well plate, growing to 30-50 % confluency next day. 2.5 µL 20 µM siRNA stock solution or 1 µL 50 µM siRNA stock solution, respectively, were diluted in 250 µL transfection medium and incubated for 20 mins (RT) with 5 µL Lipofectamine 2000 (also diluted in 250 µL Opti-MEM). The mixture was applied to the cells leading to a final siRNA concentration of 20 nmol/L. 48 hrs post transfection, SESTD1 was almost completely knocked down by 20 nM siRNA (6.6 µM each) whereas expression of an unrelated protein (GAPDH) was not altered (Figure 10).

**Table 3: List of used siRNAs.**

| **SiRNA** | **Sequence** | **Provider or SEQ ID NO:** | **catalogue number** |
|---|---|---|---|
| siGENOME Set of 4, human SESTD1, Duplex 1 | S: GAA CUU AAU CAG CAA AUU GUU | (SEQ ID NO: 8) | D-018379-01 |
| | A: 5'-P CAA UUU GCU GAU UAA GUU CUU | (SEQ ID NO: 9) | |
| siGENOME Set of 4, human SESTD1, Duplex 3 | S: GAG AGU ACA UAG AUU GGA AUU | (SEQ ID NO: 10) | D-018379-03 |
| | A: 5'-P UUC CAA UCU AUG UAC UCU CUU | (SEQ ID NO: 11) | |
| siGENOME Set of 4, human SESTD1, Duplex 4 | S: GGA UGA AAC UAG UUA AUC UUU | (SEQ ID NO: 12) | D-018379-04 |
| | A: 5'-P AGA UUA ACU AGU UUC AUC CUU | (SEQ ID NO: 13) | |
| *Silencer*R Negative Control #2 | | Ambion | 4613 |

HM1-C5Y cells transfected with 20 nM pooled SESTD-siRNA duplexes, an unspecific non-silencing control siRNA (*Silencer*R Negative Control #2, Ambion) or liposomes only were functionally tested in fluorometric [Ca²⁺]_{¡} measurements. TRPC5-independent Ca²⁺ release from internal stores after M1 and PAR activation was not significantly different between the three groups. In contrast TRPC5-mediated Ca²⁺ influx following application of carbachol or trypsin was significantly reduced in cells treated with specific SESTD1 siRNA. TRPC5-mediated Ca²⁺ influx following carbachol stimulation was reduced to 45.4 ± 2.8% (n = 14) compared to mock transfected cells or 49.6 ± 3.1% (n = 14) compared to cells transfected with control siRNA. When cells were activated with 100 nM trypsin; TRPC5-mediated Ca²⁺ influx was reduced to 51.4 ± 3.7% (n = 15) compared to mock transfected cells or 57.5 ± 4.2% (n = 15) compared to cells transfected with control siRNA.

### Example 7: Expression of SESTD1

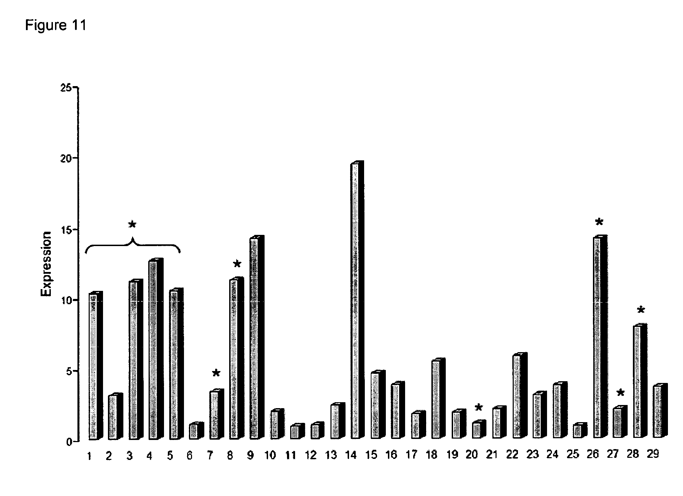

As the tissue distribution of SESTD1 has not been described, real-time quantitative PCR (TaqMan, Livak KJ, Flood SJ, Marmaro J, Giusti W, & Deetz K (1995). Oligonucleotides with fluorescent dyes at opposite ends provide a quenched probe system useful for detecting PCR product and nucleic acid hybridization. PCR Methods Appl 4, 357-362) analysis of different tissues was performed. The results showed that SESTD1 mRNA is ubiquitously expressed in human tissue (see **Figure 11****).**

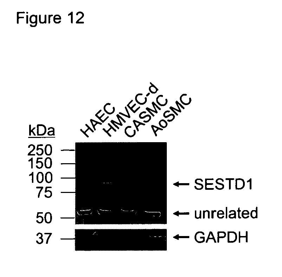

We found SESTD1 in a cDNA library made from human aorta and were interested to see in which vascular cell type the protein is expressed. Hence lysates of human primary cells were analyzed by Western blot for SESTD1 expression. SESTD1 was present both in aortic (AoSMC) and coronary (CASMC) smooth muscle cells as well as in aortic (HAEC) and microvascular (HMVEC-d) endothelial cells (Figure 12).

### Example 8: In vitro phospholipid binding

The N-terminal SEC14p-like lipid-binding domain of SESTD1 is eponym of a complete eukaryotic protein family whose members are able to specifically bind and transport different phospholipids. TRPC channel activity (Kwon Y, Hofmann T, & Montell C (2007). Integration of phosphoinositide- and calmodulin-mediated regulation of TRPC6. Mol Cell 25, 491-503) as well as many other cellular functions (e.g. cell shape and adhesion, Raucher D, Stauffer T, Chen W, Shen K, Guo S, York JD, Sheetz MP, & Meyer T (2000). Phosphatidylinositol 4,5-bisphosphate functions as a second messenger that regulates cytoskeleton-plasma membrane adhesion. Cell 100, 221-228) can be modulated by phospholipid content and distribution. Therefore, we developed assays that may be used to detect and quantify phospholipid binding to SESTD1.

### a) PIP strip phospholipid overlay assay

PIP strips (Invitrogen, Karlsruhe, Germany) are commercially available nitrocellulose membranes containing 100 pmol samples of 15 different phospholipids and a blank sample. They were blocked with different blocking buffers for PIP strips that are composed of 3% essentially fatty acid free BSA (Sigma, Munich Germany) in
a) TBST (150 mM NaCl, 10 mM Tris-HCl, 0.1% Tween 20, pH 8),
b) TBST with 0.06 µM free Ca²⁺ (150 mM NaCl, 10 mM Tris-HCl, 1 mM EGTA, 1 mM MgCl₂,0.9 mM CaCl₂, 0.1% Tween 20, pH 8)
c) TBST with 2.5 µM free Ca²⁺ (150 mM NaCl, 10 mM Tris-HCl, 1 mM EGTA, 1 mM MgCl₂, 1 mM CaCl₂, 0.1% Tween 20, pH 8)
for 1 hr (RT) and subsequently incubated with blocking buffer a) containing 500 ng/mL purified GST or blocking buffer b) or c), respectively, with 500 ng/mL GST-SESTD1 (4 hr, RT). After washing (2 times with the individual blocking buffers and subsequently once with blocking buffer a)) they were incubated at 4°C overnight with the first antibody anti-GST (1:2,000 in blocking buffer a)). Three wash steps with TBST (pH 8) were followed by 45 min incubation with the secondary HRP-conjugated anti-rabbit antibody (1:20,000 in blocking buffer a)). The membranes were washed again three times with TBST, incubated with 1 mL Lumi-LightPLUS Western blotting substrate (5 min) (Roche, Mannheim, Germany) and the chemiluminescent signals were analyzed with a Lumi Imager (Roche, Mannheim, Germany).

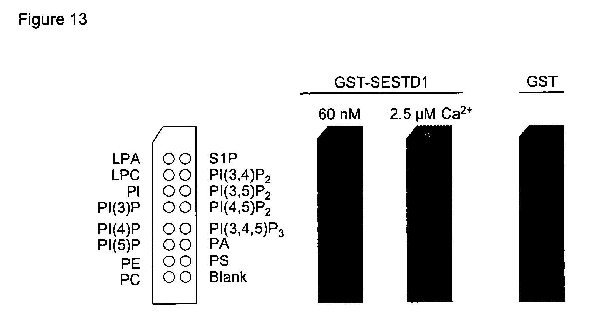

Specific binding of SESTD1 to all physiological found phosphatidyl-inositol-mono- and diphosphates (PIP, PIP₂) as well as phosphatidic acid was detected in a phospholipid overlay assay. Binding of SESTD1 to these substrates changed depending on the Ca²⁺ concentration. SESTD1 strongly bound PIPs and to a lesser extent the different PIP₂ and phosphatidic acid in presence of 60 nM Ca²⁺, the approximate physiological concentration in quiescent cells. Simulating cell activation by raising the Ca²⁺ concentration (2.5 µM) led to increased binding of PI(3,5)P₂, PI(4,5)₂, phosphatidic acid as wells as of PI(3,4)P₂, PI(3)P and-PI(4)P **(****Figure 13****).**

### b) Cova-PIP plate binding assay

The GST-tagged PH-domain of LL5-α, GST alone or GST-SESTD1 were diluted in blocking buffer a) for PIP strips (Example 8a). Cova-PIP specifity plates (Echelon, Salt Lake City, USA) loaded with 10 or 100 pmol PIPₙ per well were incubated with 100 µL protein solution per well for 3 hrs at RT. Plates were washed manually 3 times with blocking buffer a) for PIP strips before 100 µL anti-GST (1:1,000 in blocking buffer a) for PIP strips were added to each well and incubated 1 hr at RT. Plate was washed 4 times with TBST (150 mM NaCl, 10 mM Tris-HCl, 0.1 % Tween 20, pH8) using an automatic plate washer.

For dissociation-enhanced lanthanide fluorescence immunoassays (DELFIA) 100 µL secondary Eu-N1-labbeled anti-rabbit antibody (500 ng/mL in blocking buffer a) for PIP strips were added per well and incubated 1 hr at RT. Plate was washed 4 times with TBST with an automatic plate washer before 100 µL enhancer solution (Perkin Elmer, Waltham, USA) were added per well. After 20 min incubation fluorescence of the lanthanide was excited (λ_{exc} = 340 nm) and read (λ_{exc} = 620 nm) in a Tecan Ultra plate reader (Tecan, Crailsheim, Germany).

The GST-tagged PH-domain of LL5-α is suggested as a control reagent that recognizes all phosphoinositides. Therefore, the binding of GST-SESTD1 and LL5-α was first compared on Cova PIP specifity plates loaded with 10 pmol substrate per well. Whereas there was significant binding of GST-tagged PH-domain of LL5-α under the chosen conditions no significant binding of GST-SESTD1 was observed.

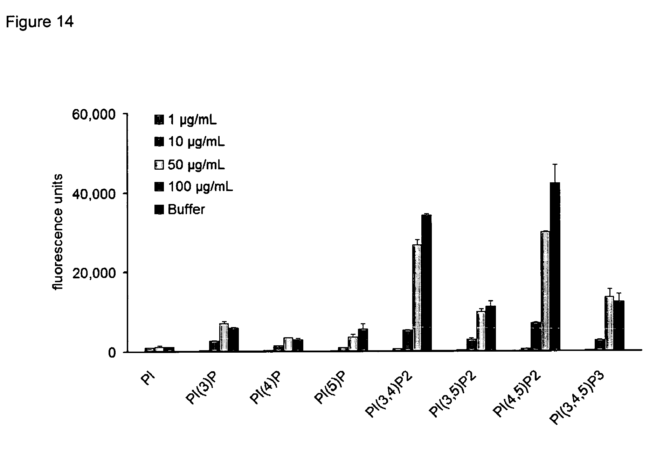

We reasoned that increasing the amount of substrate loading per well might increase the signal observed with GST-SESTD1. Hence, binding of varying amounts of SESTD1 was tested analogously using plates loaded with 100 pmol substrate per well. Indeed, SESTD1 bound specifically to all phosphoinositides with highest affinity to PI(4,5)P₂ and PI(3,4)P₂ (Figure 14).

### FIGURES

**Figure 1****: Topology of SESTD1.** The full length protein consists of 696 amino acids (expected molecular weight 79 kDa) and is composed of three structural domains. Sec 14 (aa 8-147): SEC14p-like lipid-binding domain; Spec 1 (aa 233-381), Spec 2 (aa 430-605): spectrin repeats 1 and 2.
**Figure 2****: Mapping of the SESTD1-binding site of the mTRPC4α-C-terminus.** (A) Schematic representation of the mTRPC4α C-terminus truncations screened for SESTD1 binding. (B) Yeast colonies cotransformed with truncation mutants of mTRPC4α C-terminus (bait) and full length hSESTD1 (prey) were plated on selective -Trp/-Leu/-Ade/-His agar plates. Yeast growth (bright colour) indicates interaction of the constructs.
**Figure 3****: Yeast two-hybrid assay of the interaction between the C-terminus of different TRPCs and SESTD1.** Shown are yeast colonies cotransformed with the C-terminus of the indicated TRPC channels (bait) and full length hSESTD1 (prey) plated on selective -Trp/-Leu/-Ade/-His agar plates. Yeast growth (bright colour) indicates interaction of the constructs.
**Figure 4****: GST-SESTD1 pulldown** of **mTRPC4α-C-terminus** (aa 615-974). Anti-TRPC4 (1:200) immunoblot of samples precipitated with GST-SESTD1 (lane 3) or GST (negative control, lane 4) from HEK293 cells overexpressing mTRPC4α-C terminus (aa 615-974). The blot was developed with secondary Alexa Fluor 680 goat anti-rabbit (1:2,500) antibody.
**Figure 5****: Schematic depiction of GST-SESTD1 fusion proteins.** GST fusion proteins containing different portions of SESTD1 were constructed according to the given scheme. Proteins were expressed and purified from bacteria for GST pulldown studies.
**Figure 6****: Identification of Spec 1 as interaction site in SESTD1. (A, B)** Anti-TRPC4 immunoblot of samples precipitated with the indicated GST fusion proteins or GST from HEK293 cells overexpressing mTRPC4α (A) or mTRPC4β (B). (C) Anti-TRPC5 immunoblot of a similar experiment with HEK 293 cells overexpressing mTRPC5. Blots were developed with secondary Alexa Fluor 680 goat anti-rabbit (1:2,500) antibody.
**Figure 7****: Confirmation of Spec 1 as TRPC4/5-interaction** site in SESTD1. Yeast colonies cotransformed with the mTRPC4α- or mTRPC5-C-terminus and full length SESTD1 or SESTD1-Sec 14, -Spec 1, or -Spec 2 constructs plated on -Trp/-Leu/-His/-Ade are shown. Yeast growth (bright colour) indicates interaction of the constructs.
**Figure 8****: Polyclonal SESTD1 antibodies detect endogenous and overexpressed SESTD1.** Lysates from non-transfected (N) HM1 cells and from HM1 cells transfected with HA-tagged SESTD1 (T) were probed on Western blots with anti-HA and anti-SESTD1 #147 **(A)** or anti-SESTD1 #148 **(B)** antibodies and secondary Alexa Fluor 680 goat anti-rabbit (1:2,500) antibody. Both antibodies detected HA-SESTD1 as well as endogenous SESTD1 (in non-transfected cells) as proteins with an apparent mass of about 80 kDa.
**Figure 9****: SESTD1 coimmunoprecipitates with mTRPC4β and mTRPC5.** (A) Western blot of anti-TRPC4 immunoprecipitates (P) and the corresponding lysates (L) from membranes of HM1 cells cotransfected with HA-tagged SESTD1 and FLAG-tagged mTRPC4β or pcDNA3.1. **(B)** Western blot of anti-GFP immunoprecipitates (P) and the corresponding lysates (L) from HM1 cells cotransfected with HA-tagged SESTD1 and GFP-tagged mTRPC5 or pcDNA3.1. Both blots **(A, B)** were probed with anti-SESTD1 antibody # 148 (1:5,000) and Alexa Fluor 680 goat anti-rabbit (1:2,500).
**Figure 10****: Transfection of siRNA efficiently reduces expression of SESTD1 protein in HM1-C5Y cells.** HM1-C5Y cells were transfected with 20 nM specific SESTD1 siRNA (Sp. siRNA), unspecific control siRNA (Unsp. siRNA) or liposomes only (Mock) and analyzed 48 hr post transfection. Western blots incubated with anti-SESTD1 and anti-GAPDH antibodies showed significant decrease of endogenous SESTD1 protein levels in siRNA transfected cells.
**Figure 11****: Expression of SESTD1 mRNA in human tissues.** SESTD1 mRNA expression was determined in different human tissues with qRT-PCR and normalized to expression of the housekeeping gene RPL37a. Data shown is the mean of duplicates.
   1 brain; 2 cerebellum; 3 hippocampus; 4 cortex; 5 spinal cord; 6 adrenal gland, 7 heart; 8 aorta; 9 adipose; 10 spleen; 11 bone marrow; 12 skeletal muscle; 13 skin; 14 trachea; 15 lung; 16 stomach; 17 small intestine; 18 colon; 19 liver; 20 pancreas; 21 kidney; 22 breast; 23 ovary; 24 uterus; 25 placenta; 26 testis; 27 prostate; 28 AoSMC; 29 HUVEC. Asterisks denote tissues in which significant expression of TRPC4 or TRPC5 has been reported.
**Figure 12****: SESTD1 expression in human primary cells.** Western blot of the indicated cell samples developed with anti- SESTD1 #148 (1:5,000) and secondary Alexa Fluor 680 goat anti-rabbit antibody (1:2,500). Each lane was loaded with 15 µg protein. Equal loading was verified by parallel staining of the housekeeping gene GAPDH.
**Figure 13****: SESTD1 binding of phospholipids is Ca²⁺-dependent.** GST-SESTD1 specifically bound various phosphatidylinositolphosphates (PIPs) and phosphatidic acid (PA) but not other phospholipids in a Ca²⁺ dependent manner. PIP strips (Echelon) were probed with GST-SESTD1 in blocking buffer containing 60 nM or 2.5 µM free Ca²⁺, or with GST in blocking buffer followed by anti-GST antibodies (1:2,000) and HRP-coupled goat anti-rabbit antibodies (1:20,000). Signals were detected by enhanced chemiluminescence (ECL).
**Figure 14****: Detection of SESTD1-phospholipid binding in Cova-PIP specifity plates using DELFIA.** Polystyrene microtiter wells each loaded with 100 pmol PIPₙ were incubated 3 hrs with the given concentrations GST-SESTD1 or buffer only. Bound protein was detected with anti-GST (1:1,000) and secondary Eu-N1-labbeled goat anti-rabbit antibody (50 ng/well). Lanthanide fluorescence (λ_{exc}= 340 nm, Aₑₘ = 620 nm) was measured.

## Claims

1. An isolated complex comprising
- a first protein comprising or consisting of a transient receptor potential channel (TRPC) or a functionally active variant thereof and
- a second protein comprising or consisting of the first spectrin (Spec 1) domain of SEC14 and spectrin domains 1 (SESTD1).

2. An isolated complex comprising
- a first protein comprising or consisting of a domain of a transient receptor potential channel (TRPC) or a functionally active variant thereof, the domain having the sequence LXXXXXYQEVXRNLVKRYVAAMIRXXKT (SEQ ID NO:1), wherein each X represents any amino acid residue and
- a second protein comprising or consisting of the Spec 1 domain of SESTD1.

3. An isolated complex comprising
- a first protein comprising or consisting of a transient receptor potential channel (TRPC) or a functionally active variant thereof and
- a second protein comprising or consisting of at least one protein selected from the group consisting of ankyrin repeat domain 35 (ANKRD35), apolipoprotein A-I binding protein (APOA1BP), bromodomain adjacent to zinc finger domain (BAZ1B), high-mobility group protein 2-like1 isoform b (HMG2L1), makorin RING finger protein 1 (MKRN1), pre-B-cell leukemia homeobox interacting protein 1 (PBXIP1), sarcoma antigen NY-SAR-48, spectrin alpha non-erythrocytic 1 (SPTAN1), structural maintenance of chromosomes 3 (SMC3) and talin 2 (TLN2).

4. The complex of any of claims 1 or 3, wherein the TRPC is selected from the group consisting of TRPC4, TRPC5 and TRPC6, particularly TRPC4 or TRPC5, especially TRPC4α or TRPC4β or TRPC5.

5. The complex of any of claims 1 to 4, wherein the first protein comprises the sequence LRRHHQYQEVMRNLVKRYVAAMIREAKTE (SEQ ID NO:2) or LIQNQHYQEVIRNLVKRYVAAMIRNSKTN (SEQ ID NO:3).

6. The complex of any of claims 1 to 5, wherein the TRPC is mammalian TRPC, particularly murine or human TRPC.

7. The complex of any of claims 1, 2, 4, 5 or 6, wherein the second protein comprises or consists of SESTD1.

8. The complex of any of claims 1, 2, 4, 5, 6 or 7, wherein the SESTD1 is mammalian SESTD1, particularly rat, murine or human SESTD1.

9. A test system comprising
- a first protein as defined in any of claims 1 to 6
- a second protein as defined in any of claims 1, 2, 3, 7, and 8, and
- means for detecting interaction of the first and the second protein.

10. A method for screening for a TRPC modulator comprising the steps of:
a) contacting the test system of claim 9 with a substance and
b) detecting a measurable signal upon interaction of the substance with the test system, thereby identifying the substance as a TRPC modulator.

11. The method of claim 10, wherein the influence of the substance on the interaction between the first and the second protein of the test system is detected directly.

12. The method of claim 10 or 11, wherein the first and/or the second protein comprise(s) a detectable marker.

13. The method of any of claims 10 to 12, wherein the detecting of a measurable signal involves the use of a specific antibody against the first protein and/or a specific antibody against the second protein.

14. The method of any of claims 10 to 13, wherein the TRPC modulator enhances or preferably impairs or inhibits binding of the first protein to the second protein.

15. A test system comprising
- a protein as defined as a second protein in any of claims 1, 2, 7,and 8, and
- a phospholipid, and
- means for detecting the interaction of the protein and the phospholipid.

16. The method of claim 15 wherein the phospholipide is selected from the group consisting of lysophosphatidic acid, lysophosphocholine, phosphatidyl-inositol-(3)phosphate, phosphatidyl-inositol-(4)phosphate, phosphatidyl-inositol-(5)phosphate, Phosphatidylethanolamine, Phosphatidylcholine,, Sphingosine-1-Phosphate, Phosphatidylinositol (3,4) bisphosphate, Phosphatidylinositol (3,5) phosphat,, Phosphatidylinositol (4,5) bisphosphate, Phosphatidylinositol (3,4,5) trisphosphate, phosphatidic acid, Phosphatidylserine, or a monophosphate.

17. A method for screening of a modulator of the interaction of a protein and a phospholipid comprising the steps of:
c) contacting the test system of claim 15 or 16 with a substance and
d) detecting a measurable signal upon interaction of the substance with the test system, thereby identifying the substance as a modulator of the interaction between the protein and the phospholipid.

18. The method of any of claims 10 to 17, wherein the method is used for screening for a medicament for preventing and/or treating a disease involving endothelial dysfunction.

19. The method of any of claims 10 to 17, wherein the method is used for screening for a medicament for preventing and/or treating a cardiovascular disease, cardiac hypertrophy, heart failure, ischemia, neointima hyperplasia, idiopathic dilated cardiomyopathy, hypertension, coronary syndrome, heart failure, renal failure, thrombosis, a chronic respiratory disease, asthma, chronic obstructive pulmonary disorder, idiopathic pulmonary hypertension, acute hypoxic pulmonary vasoconstriction, inflammatory occlusive disease and atherosclerosis.

20. Use of a test system according to claim 9 for the identification of a TRPC modulator.


SEQUENCE LISTING

<110>  Sanofi-Aventis Deutschland GmbH

<120>  Complexes of TRPC domains and SESTD1 domains and methods and uses
        involving the same

<130>  DE2007/050

<160>  15

<170>  PatentIn version 3.4

<210>  1
<211>  28
<212>  PRT
<213>  Artificial sequence

<220>
<223>  Consensus sequence


<220>
<221>  MISC_FEATURE
<222>  (2)..(6)
<223>  Xaa = any amino acid

<220>
<221>  MISC_FEATURE
<222>  (11)..(11)
<223>  Xaa = any amino acid

<220>
<221>  MISC_FEATURE
<222>  (25)..(26)
<223>  Xaa = any amino acid

<400>  1

Leu Xaa Xaa Xaa Xaa Xaa Tyr Gln Glu Val Xaa Arg Asn Leu Val Lys
1               5                   10                  15


Arg Tyr Val Ala Ala Met Ile Arg Xaa Xaa Lys Thr
            20                  25


<210>  2
<211>  29
<212>  PRT
<213>  Mus musculus

<400>  2

Leu Arg Arg His His Gln Tyr Gln Glu Val Met Arg Asn Leu Val Lys
1               5                   10                  15


Arg Tyr Val Ala Ala Met Ile Arg Glu Ala Lys Thr Glu
            20                  25


<210>  3
<211>  29
<212>  PRT
<213>  Mus musculus

<400>  3

Leu Ile Gln Asn Gln His Tyr Gln Glu Val Ile Arg Asn Leu Val Lys
1               5                   10                  15

Arg Tyr Val Ala Ala Met Ile Arg Asn Ser Lys Thr Asn
            20                  25

<210>  4
<211>  44
<212>  PRT
<213>  Artificial

<220>
<223>  inserted leucine zipper

<400>  4

Gly Gly Gly Ser Gly Ser Arg Met Lys Gln Ile Glu Asp Lys Leu Glu
1               5                   10                  15

Glu Ile Leu Ser Lys Leu Tyr His Ile Glu Asn Glu Leu Ala Arg Ile
            20                  25                  30

Lys Lys Leu Leu Gly Glu Arg Gly Gly Ser Gly Ser
        35                  40

<210>  5
<211>  9
<212>  PRT
<213>  Artificial

<220>
<223>  HA tag

<400>  5

Tyr Pro Tyr Asp Val Pro Asp Tyr Ala
1               5

<210>  6
<211>  15
<212>  PRT
<213>  Artificial

<220>
<223>  antigen aa 265-280 of SESTD1

<400>  6

Lys Arg Gln Gln Leu Arg His Pro Glu Met Val Thr Thr Glu Ser
1               5                   10                  15

<210>  7
<211>  16
<212>  PRT
<213>  Artificial

<220>
<223>  antigen aa 682-696 of SESTD1

<400>  7

Cys Arg Gln Arg Ser Lys Arg Thr Gln Leu Glu Glu Ile Gln Gln Lys
1               5                   10                  15

<210>  8
<211>  21
<212>  RNA
<213>  Artificial

<220>
<223>  siGENOME Set of 4, human SESTD1, Duplex 1 S

<400>  8
gaacuuaauc agcaaauugu u                                                21

<210>  9
<211>  21
<212>  RNA
<213>  Artificial

<220>
<223>  siGENOME Set of 4, human SESTD1, Duplex 1 A

<400>  9
caauuugcug auuaaguucu u                                                21

<210>  10
<211>  21
<212>  RNA
<213>  Artificial

<220>
<223>  siGENOME Set of 4, human SESTD1, Duplex 2 S

<400>  10
uauagaacca ugccaauuau u                                                21

<210>  11
<211>  21
<212>  RNA
<213>  Artificial

<220>
<223>  siGENOME Set of 4, human SESTD1, Duplex 2 A

<400>  11
uaauuggcau gguucuauau u                                                21

<210>  12
<211>  21
<212>  RNA
<213>  Artificial

<220>
<223>  siGENOME Set of 4, human SESTD1, Duplex 3 S

<400>  12
gagaguacau agauuggaau u                                                21

<210>  13
<211>  21
<212>  RNA

<213>  Artificial

<220>
<223>  siGENOME Set of 4, human SESTD1, Duplex 3 A

<400>  13
uuccaaucua uguacucucu u                                                21


<210>  14
<211>  21
<212>  RNA
<213>  Artificial

<220>
<223>  siGENOME Set of 4, human SESTD1, Duplex 4 S

<400>  14
ggaugaaacu aguuaaucuu u                                                21


<210>  15
<211>  21
<212>  RNA
<213>  Artificial

<220>
<223>  siGENOME Set of 4, human SESTD1, Duplex 4 A

<400>  15
agauuaacua guuucauccu u                                                21